# EUROPEAN PATENT SPECIFICATION

(11) **EP 2 699 671 B1**
(45) Date of publication and mention of the grant of the patent: **10.07.2019**
(21) Application number: 12774145.2
(22) Date of filing: 11.04.2012
(51) Int. Cl.: A61K 35/14, A61K 35/28, C07K 16/00

(54) **ANTI-INFLAMMATORY AGENTS**
ENTZÜNDUNGSHEMMENDE WIRKSTOFFE
AGENTS ANTI-INFLAMMATOIRES

(30) Priority: 21.04.2011 US 201161477935 P
(43) Date of publication of application: 26.02.2014
(73) Proprietor: The Rockefeller University, New York, NY 10021 (US)
(72) Inventor: RAVETCH, Jeffrey, V., New York, NY 10022 (US); ANTHONY, Robert, Cambridge, MA 02138-6802 (US); KOBAYASHI, Toshihiko, Tokyo 110-0008 (JP); WERMELING, Fredrik, New York, NY 1005 (US)
(74) Representative: Zacco GmbH
(86) International application number: PCT/US2012/033095
(87) International publication number: WO 2012/145209

(56) References cited:
- WO-A2-2011/031736
- US-A1- 2009 004 179
- PRICOP ET AL: "Differential modulation of stimulatory and inhibitory Fc gamma receptors on human monocytes by Th1 and Th2 cytokines.", THE JOURNAL OF IMMUNOLOGY, vol. 166, no. 1, 1 January 2001 (2001-01-01), pages 531-7, XP055137924, ISSN: 0022-1767
- SCHMITZ J ET AL: "IL-33, an interleukin-1-like cytokine that signals via the IL-1 receptor-related protein ST2 and induces T helper type 2-associated cytokines", IMMUNITY, CELL PRESS, US, vol. 23, no. 5, 1 November 2005 (2005-11-01), pages 479-490, XP002432892, ISSN: 1074-7613, DOI: 10.1016/J.IMMUNI.2005.09.015
- A. S. W. TJON ET AL: "Intravenous Immunoglobulin Treatment in Humans Suppresses Dendritic Cell Function via Stimulation of IL-4 and IL-13 Production", THE JOURNAL OF IMMUNOLOGY, vol. 192, no. 12, 15 June 2014 (2014-06-15), pages 5625-5634, XP055137754, ISSN: 0022-1767, DOI: 10.4049/jimmunol.1301260
- ANTHONY ET AL.: 'Identification of a receptor required for the anti-inflammatory activity of IVIG.' PROC NAT ACAD SCI vol. 105, no. 50, 16 December 2008, pages 19571 - 19578, XP055053128
- SMITS ET AL.: 'Selective probiotic bacteria induce IL-10-producing regulatory T cells in vitro by modulating dendritic cell function through dendritic cell-specific intercellular adhesion molecule 3 -grabbing nonintegrin.' J ALLERGY CLIN IMMUNOL. vol. 115, no. 6, June 2005, pages 1260 - 1267, XP004919073
- ANTHONY ET AL.: 'Intravenous gammaglobulin suppresses inflammation through a novel TH2 pathway.' NATURE vol. 475, no. 7354, 19 June 2011, pages 110 - 113, XP055132104

## Description

### FIELD OF INVENTION

This invention relates to a method for measuring the anti-inflammatory activity of a DC-SIGN-binding composition, and is defined in the claims.

### BACKGROUND OF INVENTION

Inflammatory disorders, including autoimmune diseases, are disorders involving abnormal activation and subsequent migration of white blood cells to affected areas of the body. These conditions encompass a wide range of ailments that affect the lives of millions of people throughout the world. Although various treatments are presently available, many possess significantly side effects or are not very effective in alleviating all symptoms. At the same time, few tests exist that reliably identify or evaluate such treatments. Thus, there are needs for anti-inflammatory agents for treating inflammatory disorders and needs for methods of identifying and evaluating such agents.

Intravenous immunoglobulin (IVIG) is a preparation containing pooled IgG purified from the plasma of blood donors and high dose IVIG is a widely used therapeutic preparation. It is administered at high doses (1-2g/kg) for the suppression of autoantibody triggered inflammation in a variety of clinical settings (Nimmerjahn et al. Annu Rev Immunol 26, 513-533 (2008)). This anti-inflammatory activity of IVIG is triggered by a minor population of IgG Fcs, with glycans terminating in α2,6 sialic acids (sFc) that target myeloid regulatory cells expressing the lectin Dendritic Cell-Specific ICAM-3 Grabbing Non-Integrin (DC-SIGN; Kaneko et al. Science 313, 670-673 (2006); Anthony et al. Science 320, 373-376 (2008); and Anthony et al. Proc Natl Acad Sci U S A13 105, 19571-19578 (2008)).

The present invention addresses and meets the above-mentioned needs by identifying cytokines and cells that recapitulate the anti-inflammatory activity of IVIG, thus allowing for methods and assays useful in identifying and evaluating agents for treating inflammatory disorders.

WO2011/031736 discloses methods of identifying anti-inflammatory compounds.

### SUMMARY OF INVENTION

The present invention is directed to a method for measuring the anti-inflammatory activity of a DC-SIGN-binding composition comprising a polypeptide containing a Fc region that has a N-linked biantennary oligosaccharide having a terminal sialic acid connected to galactose by an α 2,6 linkage. The method includes (a) administering a DC-SIGN-binding composition comprising a polypeptide containing a Fc region that has a N-linked biantennary oligosaccharide having a terminal sialic acid connected to galactose by an α 2,6 linkage to a mouse; and (b) measuring the expression level of IL-33 mRNA in splenocytes of the mouse, wherein an increase in the expression level of IL-33 mRNA in splenocytes of the mouse is indicative of the anti-inflammatory activity of the DC-SIGN-binding composition. In one example, the expression level of IL-33 mRNA is obtained by qPCR. Again, the DC-SIGN-binding composition is one having a polypeptide containing a Fc region that has a N-linked biantennary oligosaccharide having a terminal sialic acid connected to galactose by an α 2,6 linkage, such as an IVIG preparation.

The details of one or more embodiments of the invention are set forth in the description below. Other features, objects, and advantages of the invention will be apparent from the description and from the claims.

### DESCRIPTION OF DRAWINGS

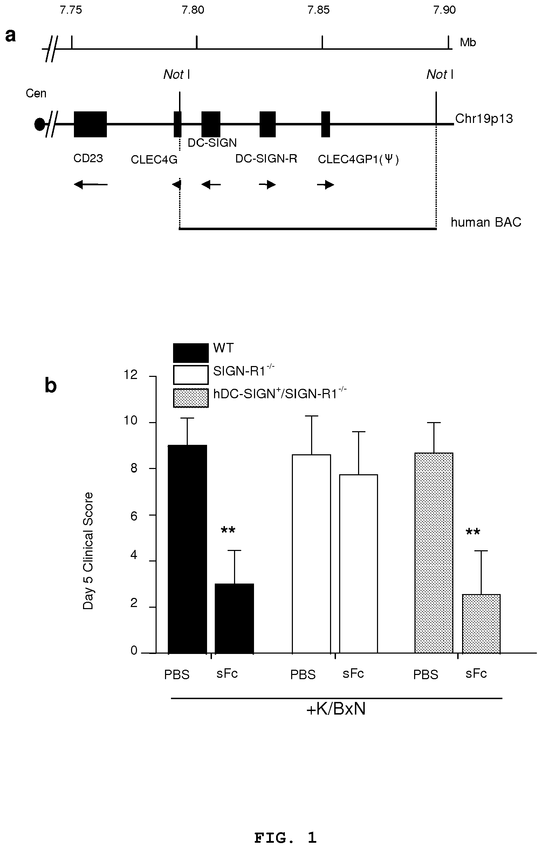

FIGs. 1a-d are diagrams showing that human DC-SIGN conveyed the anti-inflammatory response of sFc. (a) A map of the BAC clone of the human chromosome 19, which contains DC-SIGN, DC-SIGN-R, and CLEC4GP1 genes, used to generate hDC-SIGN⁺ mice (cen, centromere). (b), Wild type (WT, black bars), SIGN-R1^{-/-} (white bars), or hDC-SIGN⁺/SIGN-R1^{-/-} (gray bars) mice were administered K/BxN sera, some of which received sFc, and footpad swelling was monitored over the next several days. Means and standard deviations of day 5 clinical scores are plotted; **denotes p<0.001 compared to K/BxN treated control as determined by a Fisher LSD posthoc test. (c), WT and hDC SIGN⁺ bone marrow-derived macrophages (BMMΦ) were pulsed with asialoFc (1.5mg/ml, black bars) or sFc (1.5mg/ml, white bars) *in vitro,* recovered, and administered to WT recipient mice treated with K/BxN sera. Means and standard deviations of day 5 clinical scores are plotted; *p<0.05 as determined by Tukey's posthoc test. (d) sFc treated hDC-SIGN⁺ BMMΦ were transferred to SIGN-R1^{-/-} and FcγRIIB^{-/-} recipients. Control (PBS, black bars) and recipient mice (white bars) received K/BxN sera, and footpad swelling was monitored over the next several days. Means and standard deviations of day 7 clinical scores are plotted; **p<0.001 as determined by Tukey's posthoc test.

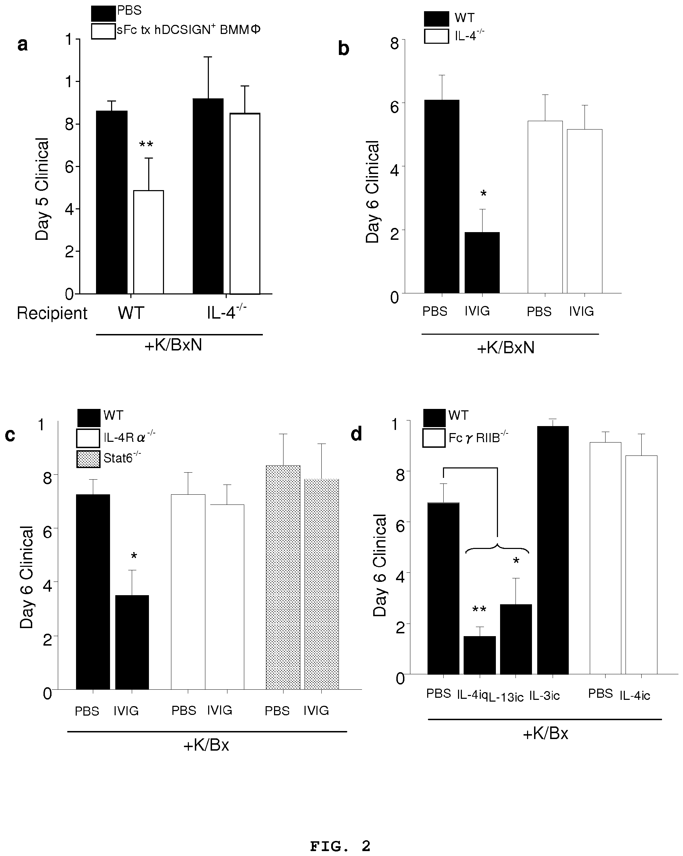

FIGs. 2a-d are diagrams showing IL-4 requirements of sFc anti-inflammatory activity, (a) sFc treated hDC-SIGN⁺ BMMΦ were administered to wild type or IL-4^{-/-} recipient mice. Engrafted (white bars) and control (PBS, black bars) mice were administered K/BxN sera, and footpad swelling monitored. Means and standard deviations of day 5 clinical scores are plotted; **p<0.002 as determined by Fisher LSD posthoc test. (b) WT (black bars) and IL-4^{-/-} (white bars) mice were treated with K/BxN sera and IVIG. Day 6 clinical score means and standard deviations are plotted; *p<0.01 as determined by Mann-Whitney's U test. (c) WT (black bars), IL-4Rα^{-/-} (white bars), or Stat6^{-/-} mice (gray bars) were given K/BxN sera and IVIG. Means and standard deviation of day 6 clinical scores are plotted; *p<0.01 as determined by Tukey's posthoc test. (d) WT (black bars) and FcγRIIB^{-/-} mice (white bars) were administered cytokine immune complexes (IL-4ic, IL-3ic, IL-13ic) and K/BxN sera. Means and standard deviations of day 6 clinical scores are plotted; *p<0.01, **p<0.001 as determined by Mann Whitney's U test.

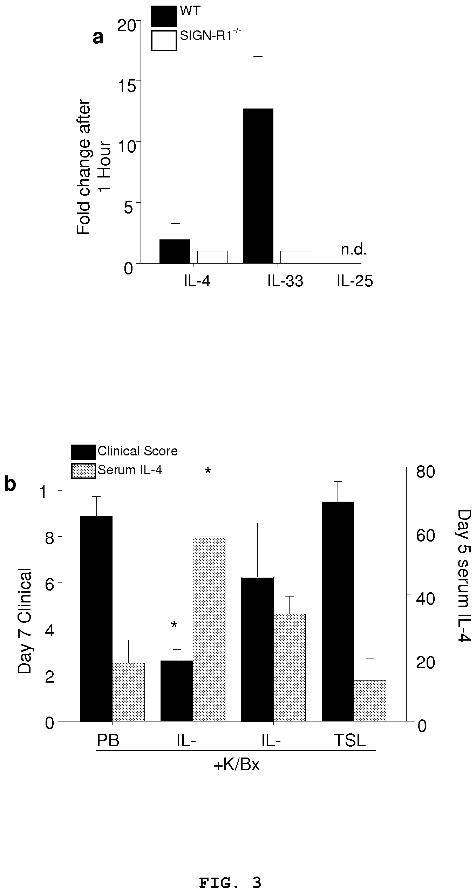

FIGs. 3a-f are diagrams showing that IL-33 triggered IL-4 anti-inflammatory activity, (a) WT (black bars) or SIGN-R1^{-/-} mice (white bars) were administered IVIG, and spleens recovered after 1 hour, and expression of Th2 cytokines was determined by qPCR; n.d., not detected. (b) WT mice were administered K/BxN sera and daily injections of PBS, IL-33, IL-25, or TSLP. Means and standard deviations of day 7 clinical scores (black bars) and systemic IL-4 levels (gray bars) are plotted; *p<0.05 as determined by Tukey's posthoc test. (c) WT (black bars) or IL-4Rα^{-/-} (white bars) mice received K/BxN sera and PBS, IL-4ic, or IL-33. Means and standard deviations of day 5 clinical scores are plotted; **p<0.001 as determined by Tukey's posthoc test. (d) hDC-SIGN⁺/SIGN-R1^{-/-} mice were treated with K/BxN sera, sFc, and α-IL-33Rα. Means and standard deviations of day 5 clinical scores are plotted; **p<0.001 as determined by Fisher LSD posthoc test. (e) hDC-SIGN⁺ BMMΦ were treated *in vitro* with sFc, and administered to K/BxN treated WT mice, some of which received α-IL-33Rα. Means and standard deviations of day 5 clinical scores are plotted; *p<0.05 as determined by Tukey's posthoc test. (f) WT mice were administered PBS, IL-4, IL-33, or IL-25 and surface expression of FcγRIIB was examined by FACS 24 hours later. Mean fluorescence intensities (MFI) of FcγRIIB expression on monocytes (CD11b⁺ Ly6G⁻) recovered from bone marrow are plotted; **denotes p<0.01 as determined by Tukey's posthoc test.

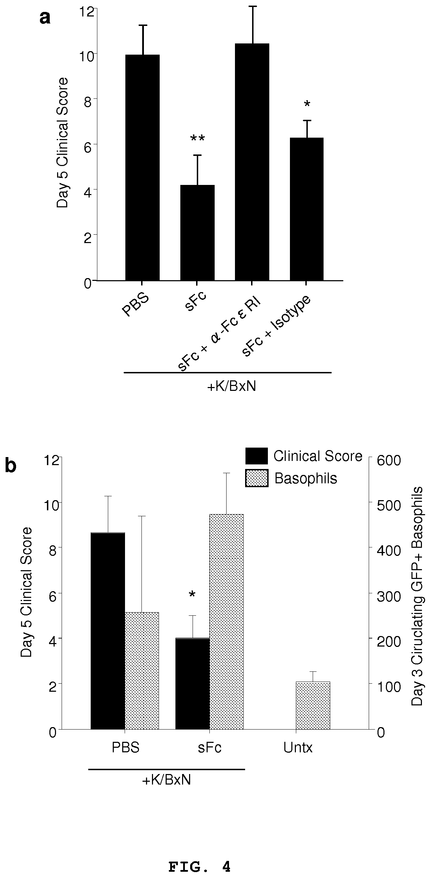

FIGs. 4a-d are diagrams showing anti-inflammatory activity mediated by basophils. (a) hDC-SIGN⁺/SIGN-R1^{-/-} mice were treated with K/BxN sera and sFc. Some mice were also treated with basophil-depleting α-FcεRI or an isotype control. Means and standard deviations of day 5 clinical scores are plotted. **p<0.001, *p<0.05 as determined by a Fisher LSD posthoc test. (b) 4get BALB/c mice were administered K/BxN sera, some of which received sFc. Means and standard error of day 3 circulating GFP⁺ basophils (gray bars, DX5⁺ FcεRI⁺) and day 5 clinical scores (black bars) are plotted; *p<0.05 as determined by Tukey's posthoc test. (c) Expanded PBS or IL-33 treated basophils (DX5⁺ FcεRI⁺ c-Kit⁻) were sorted and administered to recipient mice treated with K/BxN sera. PBS-treated and IVIG treated mice served as controls, and means and standard deviation are plotted. (d) Basophils were sorted from IL-33-treated FcγRIIB^{-/-} mice and administered to WT recipients challenged with K/BxN sera. Mean clinical scores (black) and serum IL-6 levels (gray) and standard error are plotted. *denotes p<0.05 as determined by Mann Whitney's U test.

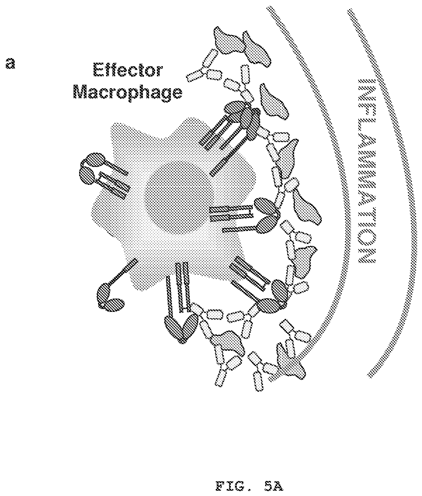

FIGs. 5a-b are diagrams showing anti-inflammatory activity of sFc. (a) Autoantibody immune complexes crosslink activating Fc receptors, promoting activation of macrophages, and inflammation associated with autoantibody mediated autoimmune disease. (b) Following administration of IVIG, antibodies with sialylated IgG Fcs bind DC-SIGN⁺ myeloid-derived cells promoting IL-33 expression, which activates FcεRI⁺ innate leukocytes to produce IL-4. This cytokine promotes upregulation of FcRIIB on macrophages, thereby increasing the activation threshold required to trigger inflammation.

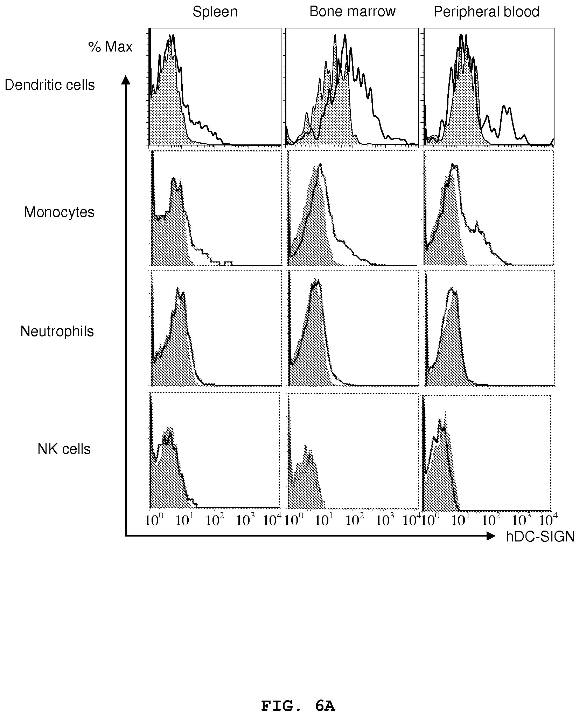

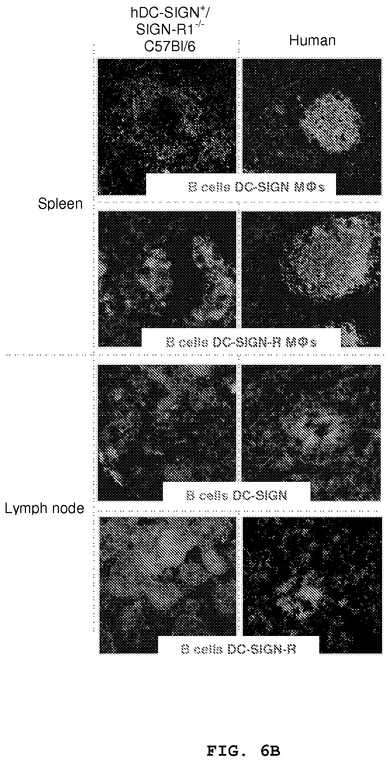

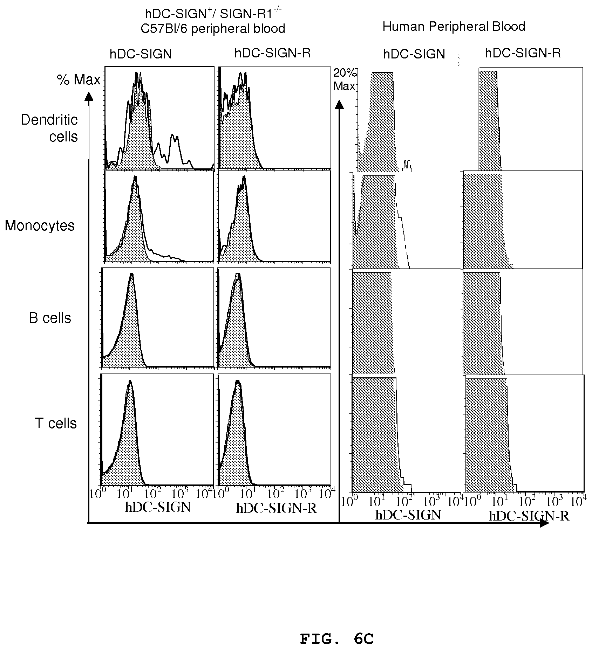

FIGs. 6a-c are diagrams and photographs showing characterization of hDC-SIGN⁺ mice. (a) FACS analysis of leukocytes recovered from spleen, bone marrow, and peripheral blood of hDC-SIGN expression in hDC-SIGN⁺/SIGN-R1^{-/-} (white histograms) and WT control (gray histograms) mice on dendritic cells (CD11c⁺ I-A^{b+}), monocytes (CD11b⁺ Ly6G⁻), neutrophils (CD11b⁺ Ly6G^{hi}), and NK cells (CD19⁻, CD3ε⁻, NKp46⁺). (b) Expression patterns of hDC-SIGN and hDC-SIGN-R was compared between hDC-SIGN⁺/SIGN-R1^{-/-} mouse and human lymphoid tissues. Spleen and lymph node cryosections were stained with α-hDC-SIGN or α-hDC-SIGN-R (red) in combination with B cell markers (green, α-B220 for mouse, α-CD20 for human) or macrophage markers (blue, α-F4/80 for mouse, α-CD68 for human) and visualized by fluorescence microscopy. (c) hDC-SIGN and hDC-SIGN-R expression was compared on leukocytes recovered from hDC-SIGN⁺/SIGN-R1^{-/-} mice and human peripheral blood. Mouse leukocytes were stained for dendritic cells (CD11c⁺ I-Ab⁺), monocytes (CD11b⁺ Ly6G⁻), B cells (CD19⁺), and T cells (CD3^{ε+}), while human leukocytes were stained for dendritic cells (CD11c⁺ HLA-DR⁺), monocytes (CD14⁺ CD16^{dim} CD19⁻ CD3⁻ CD56⁻), B cells (CD19⁺), and T cells (CD3⁺).

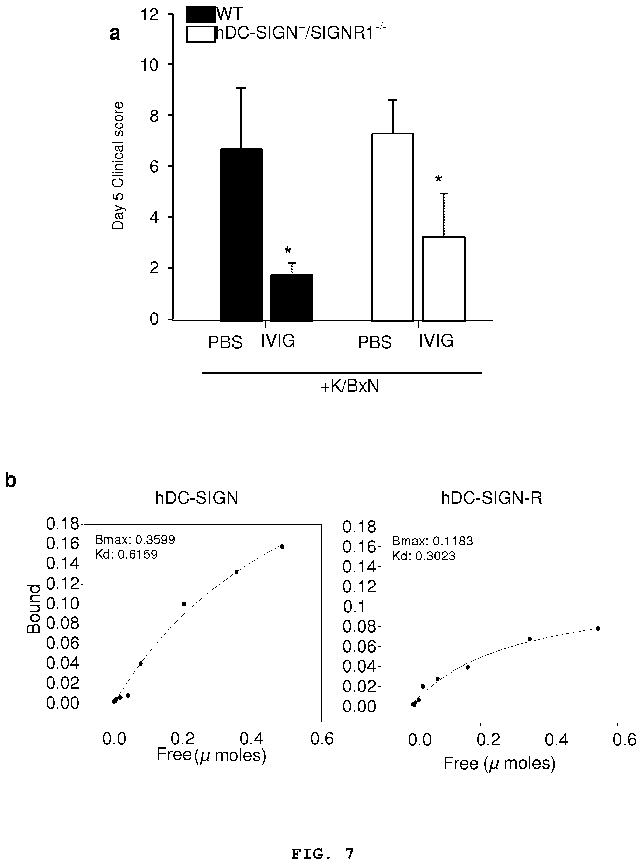

FIGs. 7a-d are diagrams showing that hDC-SIGN, but not hDC-SIGN-R, was required from IVIG anti-inflammatory activity, a, Wild type (WT, black bars) and hDC-SIGN⁺/SIGN-R1^{-/-} were administered IVIG and K/BxN sera, and footpad swelling monitored over several days. Means and standard deviations of day 5 clinical scores are plotted. (b) Saturation binding experiments were performed using cell lines that expressed hDC-SIGN or hDC-SIGN-R to determine their affinities for sFc (inset). (c) Wild type (black bars), SIGN-R1^{-/-} (white bars) and CD11c-hDC-SIGN/SIGN-R1^{-/-}(gray bars) mice were treated with K/BxN sera and IVIG, and footpad swelling monitored over several days. Means and standard deviations of day 5 clinical scores are plotted; *p<0.01 as determined by Tukey's posthoc test. (d) hDC-SIGN⁺/SIGN-R1^{-/-} BAC tg mice were administered K/BxN, IVIG, and 125ug of α-hDC-SIGN or mouse IgG2a isotype control, and footpad swelling monitored. Day 5 clinical score means and standard deviations of 4 mice per group are plotted; *p<0.05 as determined by Tukey's posthoc test.

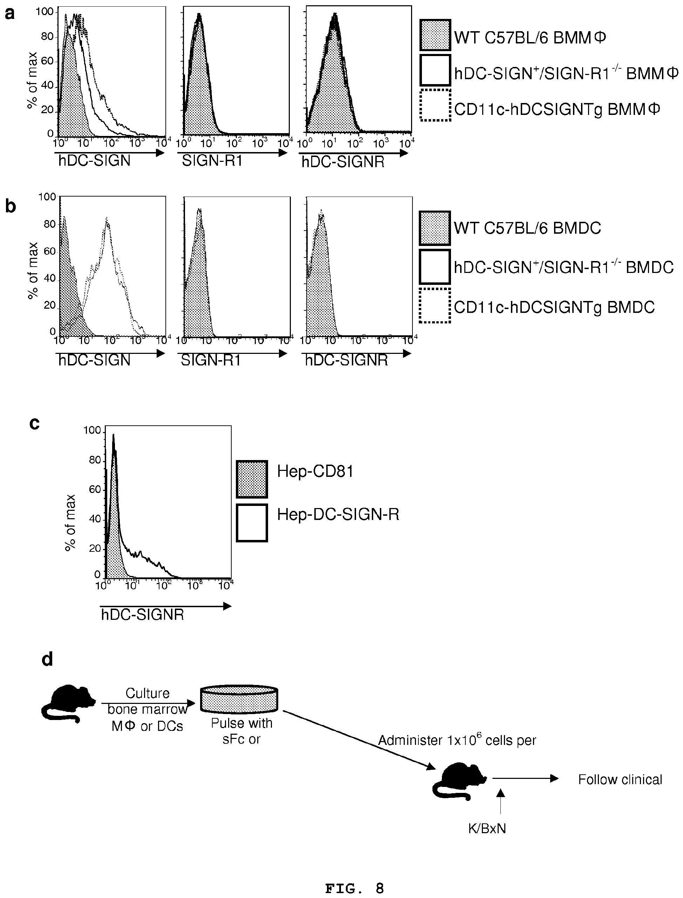

FIGs. 8a-f are diagrams showing that hDC-SIGN+ cells transferred anti-inflammatory activity. hDC-SIGN, SIGN-R1, and hDC-SIGN-R expression was compared on mature BMMΦ (a) and bone marrow-derived dendritic cells (BMDC, (b)) from wild type mice (gray fill), hDC-SIGN⁺ mice (white fill), and CD11c-hDC-SIGN mice (dotted line) by FACS. (c) Hep-hDC-SIGN-R cells (white histograms) were stained for hDC-SIGN-R expression and compared to Hep-CD81 cells (gray histogram) to validate the α-hDC-SIGN-R staining. (d) Schematic representation for BM-derived cell transfer experiments. Bone marrow progenitors were cultured into mature macrophages or dendritic cells. Differentiated cells were replated, pulsed with Fcs for 30 minutes, recovered and washed, and administered to mice that were then treated with K/BxN sera. BMMΦ (e) or BMDC (f) from WT and hDC-SIGN+ mice were pulsed with BSA (15mg/ml, blakc bars) or IVIG (15mg/ml, white bars), and transferred to WT recipient mice, which then received K/BxN sera. Footpad swelling was monitored over the next several days. Means and standard deviation of day 7 clinical scores are plotted; *p<0.05 as determined by Tukey's posthoc test.

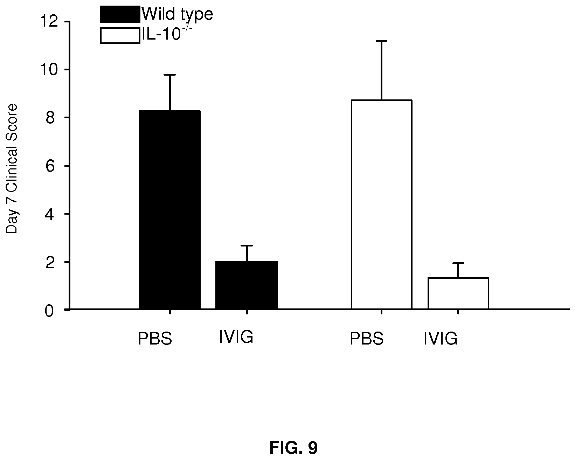

FIG. 9 is a diagram that IL-10 was not required for the anti-inflammatory activity of IVIG. WT (black bars) and IL-10^{-/-} mice (white bars) were administered K/BxN sera, some of which received IVIG, and footpad swelling monitored over the next several days. Means and standard deviations of day 7 clinical scores of 4-5 mice per group are plotted.

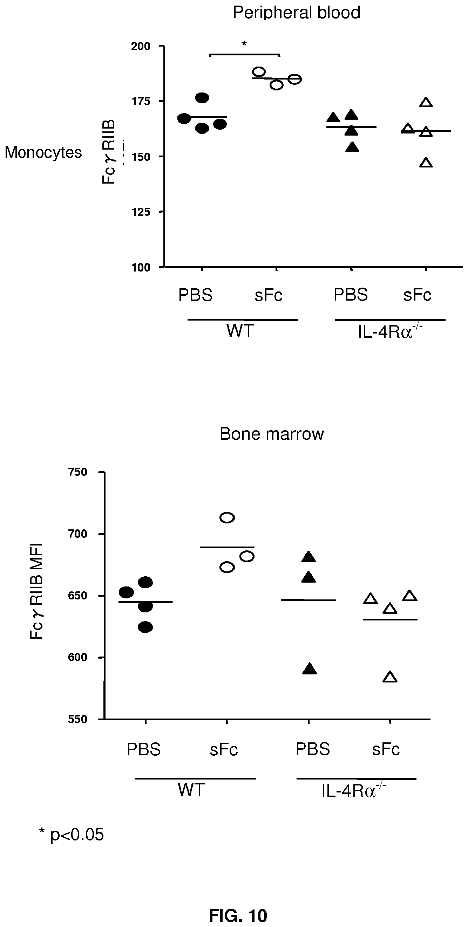

FIG 10 is a set of diagrams showing IL-4 receptor requirement for sFc-triggered FcyRIIB upregulation. WT or IL-4Rα^{-/-} were administered PBS or sFc and one day later, monocytes were recovered from peripheral blood and bone marrow, and surface expression of FcyRIIB was determined by FACS. Mean fluorescence intensities (MFI) are plotted; *p<0.05 as determined by ANOVA followed by a Tukey's posthoc test.

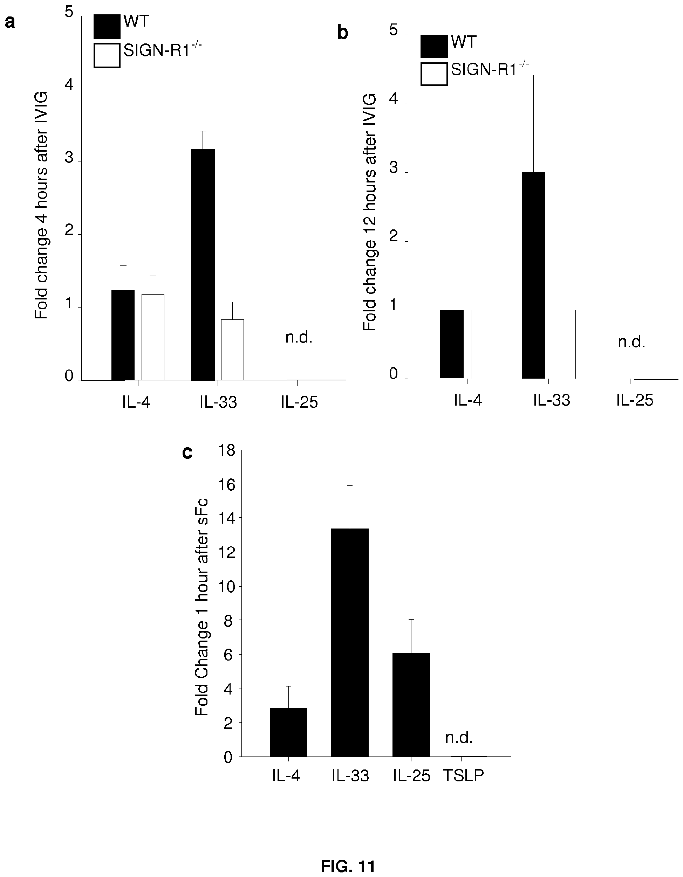

FIGs. 11a-f are diagrams showing analysis of IL-33 anti-inflammatory activity. qPCR was performed on spleen samples recovered from WT and SIGN-R1^{-/-} mice administered IVIG 4 hours (a) and 12 hours (b) earlier using cDNA synthesized from total spleen RNA to examine Th2 cytokine gene induction. (c) Spleens from sFc treated mice were recovered 1 hour later, and qPCR was performed as described herein. (d) Mice were administered K/BxN along with PBS, 400ng IL-33, 400ng IL-25, 800ng IL-25, biotinylated IL-13ic on day 0, or biotinylated IL-13ic on day 3. Also, on day 3, all mice (but IL-13ic treated groups) were administered 10µg of biotinylated α-IL-13. All mice were bled the next day, and systemic IL-13 levels were determined. (e) Wild type (black bars) or IL-4^{-/-} (gray bars) mice were administered PBS, IL-4ic, or IL-33 and K/BxN sera and monitored, suggesting IL-13 induced by IL-33 (shown in d) is sufficient to increase FcyRIIB surface expression and attenuate inflammation. Means and standard deviations of day 5 clinical scores from four mice are plotted. *p<0.05 as determined by ANOVA followed by a Tukey's posthoc test. (f) K/BxN treated mice were administered IVIG or IL-4ic, some of which received α-IL-33Rα. Means and standard deviation of day 5 clinical scores are plotted. α-IL-33Rα treatment obscured IVIG protection of K/BxN inflammation, but did not affect IL-4ic protection, indicating IL-33 and the IL-33Rα act downstream of IVIG, but upstream of IL-4.

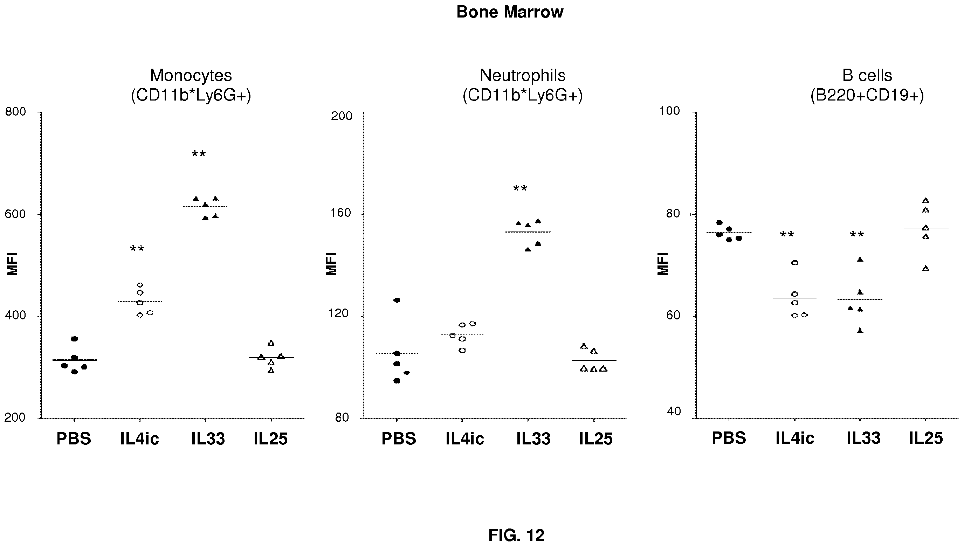

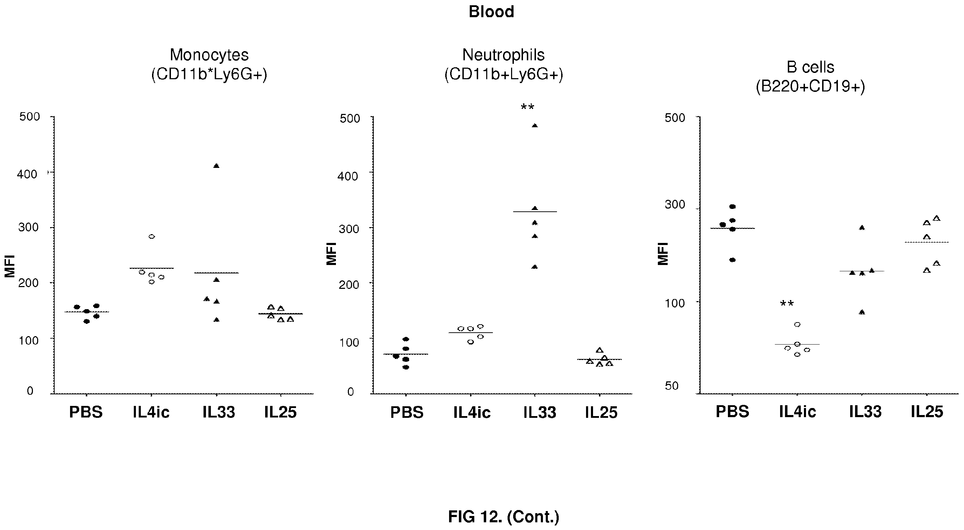

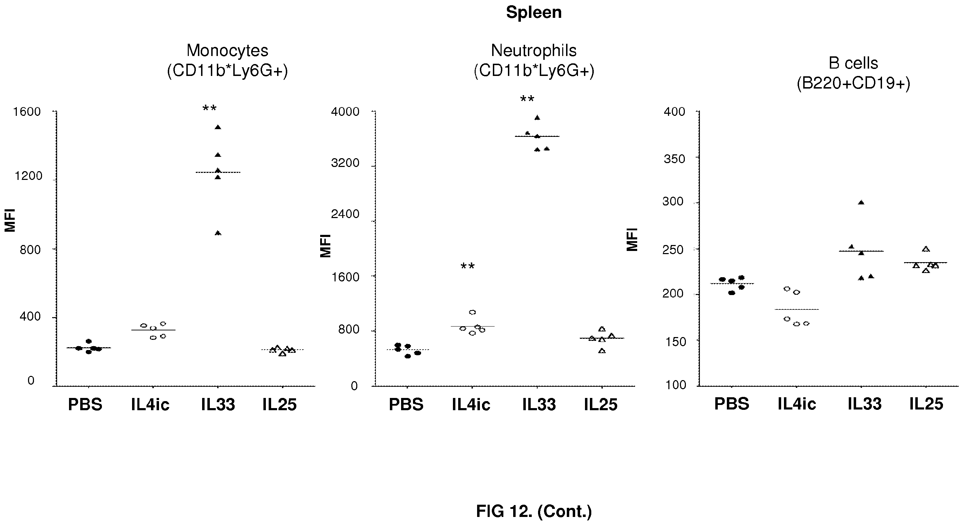

FIG. 12 is a set of diagrams showing that exogenous IL-4 and IL-33 up-regulated monocyte surface expression of FcyRIIB. Wild type mice were administered PBS (black circles), IL-4ic (white circles), IL-33 (black triangles), or IL-25 (white triangles), and the next day, monocytes (CD11b⁺ Ly6G⁻), neutrophils (CD11b⁺ Ly6G⁺) and B cells (B220⁺ CD19⁺) from bone marrow, spleen, and blood subjected to FACS. Mean fluorescence intensities (MFI) of surface FcyRIIB staining of 5 mice per group are plotted. **p<0.01 as determined by Tukey's posthoc test.

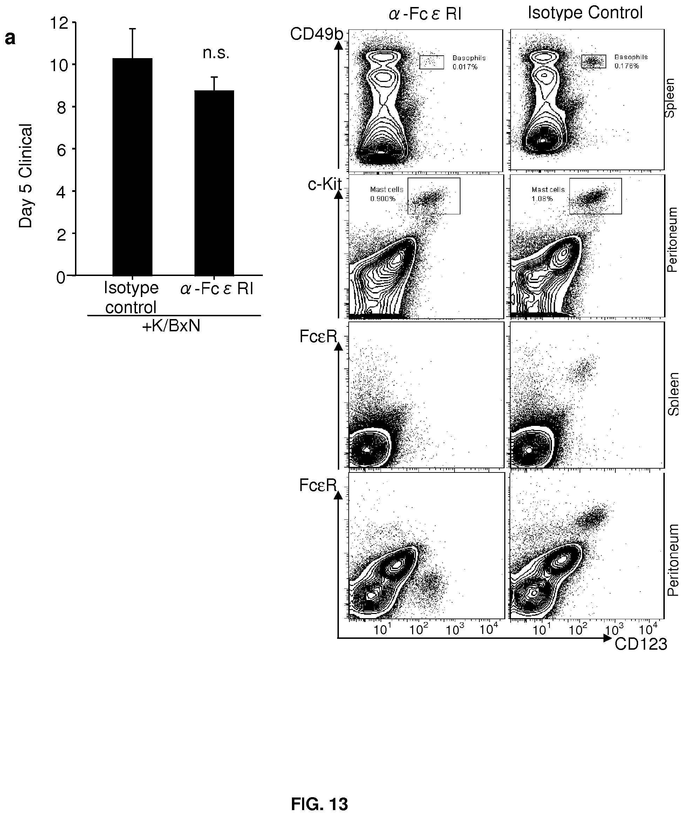

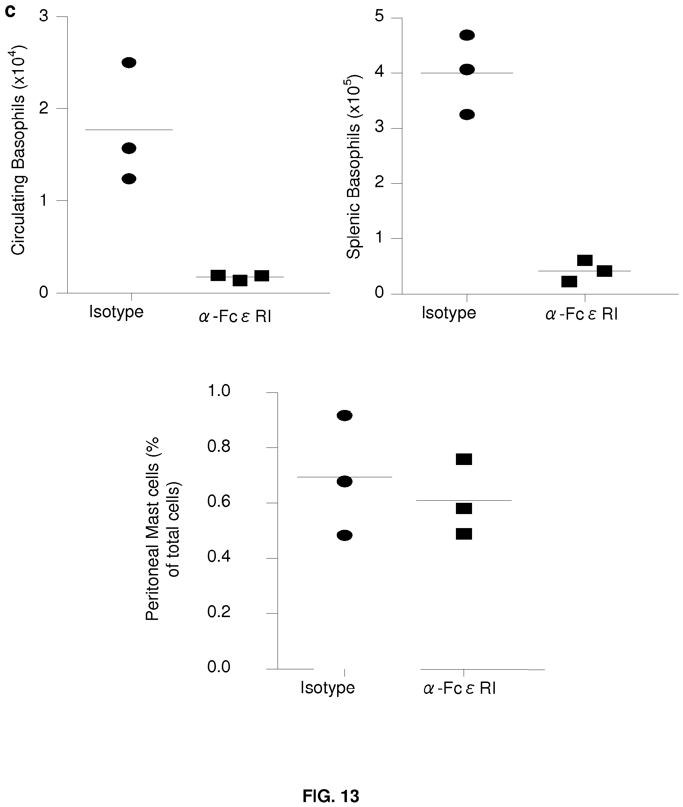

FIGs. 13a-c are diagrams showing selective depletion of basophils by α-FcεRI treatment, (a) Wild type mice received K/BxN sera and α-FcεRI or isotype control antibody, and footpad swelling was monitored. Means and standard deviations of day 5 clinical scores are plotted; n.s. (not significant) as determined by Tukey's posthoc test. (b) Basophil-depletion was evaluated in mice treated with α-FcεRI or isotype control antibody by FACS. Representative basophil (CD49b⁺ CD123⁺) and mast cell (cKit⁺ CD123⁺) percentages are inset, showing basophils but not mast cells are depleted following α-FcεRI treatment. FcεRI staining is, however, blocked on peritoneal mast cells in α-FcεRI treated but not isotype control treated mice. (c) Basophil (CD49b⁺ CD123⁺) and mast cell (c-Kit⁺ CD123⁺) numbers in α-FcεRI (black squares) or isotype control (black circles) treated mice were quantified by FACS and plotted.

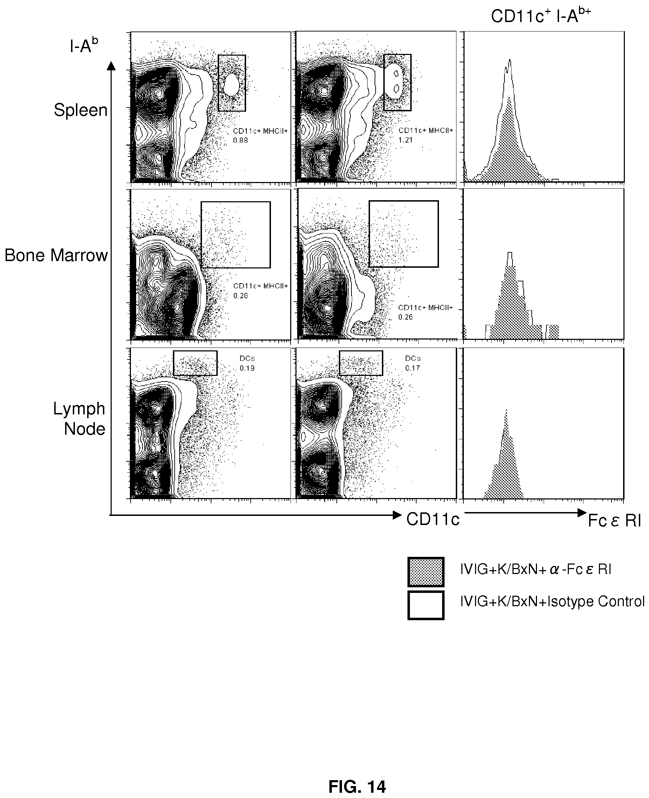

FIG. 14 is a set of histograms showing that dendritic cells were unaffected by α-FcεRI treatment.

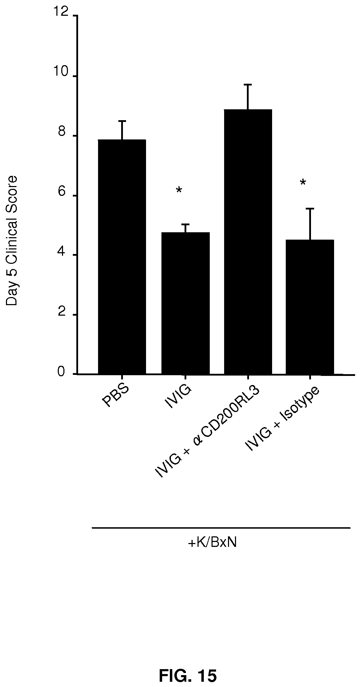

FIG. 15 is diagram showing that α-CD200RL3 treatment obscured IVIG anti-inflammatory activity. Wild type mice were treated with K/BxN sera and IVIG (1g/kg). Some of the mice were treated with α-CD200R3 antibody or rat IgG1 isotype control. Footpad swelling was monitored; mean and standard deviation of day 5 clinical scores of 5 mice per group are plotted; *p<0.05 as determined by Fisher LSD posthoc test.

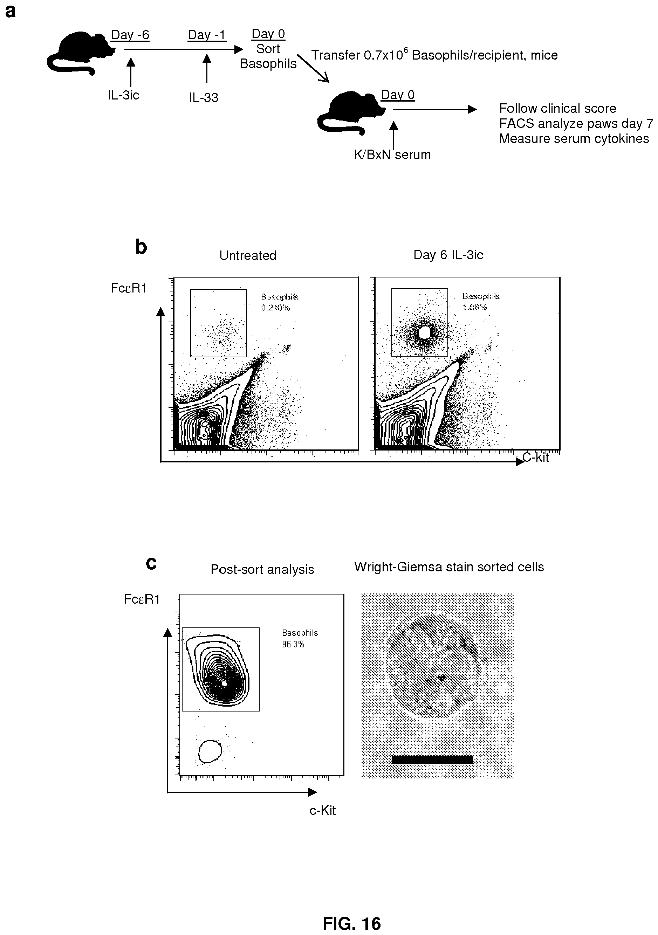

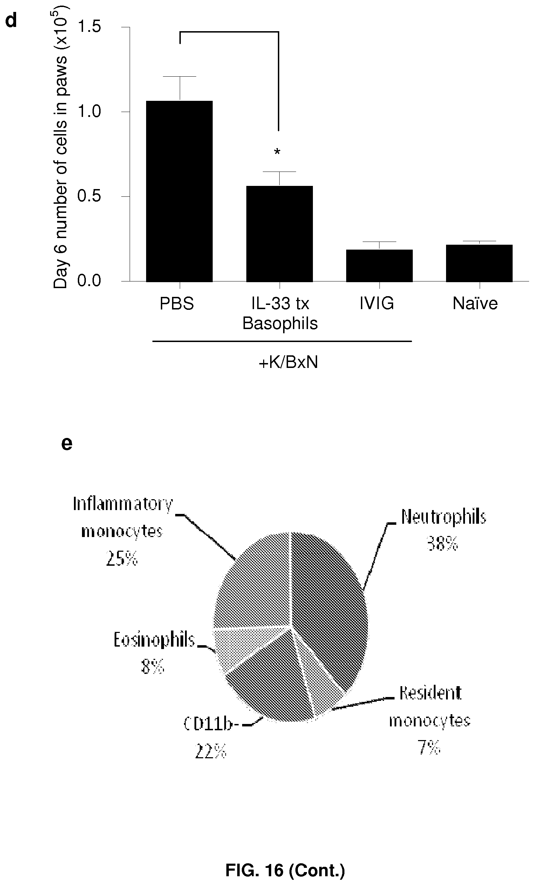

FIGs. 16a-e are diagrams and a photograph showing that IL-33-primed basophils transferred protection in the K/BxN model. (a) Schematic of basophil transfer procedure. (b) Representative FACS plot showing basophil expansion (FcεRI⁺ cKit⁻) by IL-3ic. (c) FACS sort purity of expanded basophils was assessed by FcεRI⁺ DX5⁺ cKit⁻ cells, and cell morphology was characterized by staining sorted cells after cytospin treatment using a Wright-Giemsa stain; scale bar 10um. (d) The total number of leukocytes in the paws of mice treated with K/BxN sera and PBS, IL-33 treated FcγRIIB^{-/-} basophils, or IVIG was compared to untreated (naive) mice. Means and standard deviation of 4 mice per group are plotted. (e) The relative percentages of leukocytes in K/BxN inflamed paws are plotted in a pie chart. The relative percentages were consistent and did not reflect differences in clinical score, however the total number of infiltrating cells (as shown in d) was reflective of clinical score.

### DETAILED DESCRIPTION OF THE INVENTION

This invention is based, at least in part, on unexpected discoveries of a novel DC-SIGN-Th2 pathway initiated by IVIG/sFc and discoveries of cytokines and immunosuppressive cells that recapitulate the anti-inflammatory activity of IVIG/sFc.

IVIG, as a therapeutic preparation, has been approved for the treatment of patients suffering from a number of autoimmune diseases, including immune-mediated thrombocytopenia, chronic inflammatory demyelinating polyneuropathy, and Guillain-Barre syndrome, as well as other autoimmune disorders. It is known that administration of IVIG mediates anti-inflammatory activities through interactions mediated by its Fc fragment. See Anthony et al. Proc Natl Acad Sci U S A13 105, 19571-19578 (2008) and US Patent No. 7,846,744.

As disclosed herein, to characterize the response triggered by IVIG, humanized DC-SIGN mice (hDC-SIGN) were generated. It was demonstrated that the anti-inflammatory activity of IVIG can be recapitulated by the transfer of bone marrow-derived sFc-treated hDC-SIGN⁺ macrophages or dendritic cells into naive recipients. Furthermore, sFc administration results in production of IL-33, which, in turn, induces expansion of IL-4 producing basophils that promote increased expression of the inhibitory Fc receptor, FcyRIIB, on effector macrophages. Systemic administration of the Th2 cytokines IL-33 or IL-4 upregulates FcyRIIB on macrophages, and suppresses serum-induced arthritis. Consistent with these results, transfer of IL-33-treated basophils suppressed serum-induced arthritic inflammation. This novel DC-SIGN-Th2 pathway initiated by an endogenous ligand, sFc, provides an intrinsic mechanism for maintaining immune homeostasis that can be manipulated to provide therapeutic benefit in autoimmune diseases.

Listed below are the human and mouse polypeptide sequences of IL-33 and IL-4
SEQ ID NO: 1 (the full-length polypeptide sequence of human IL-33):
SEQ ID NO: 2 (the full-length polypeptide sequence of mouse IL-33):
SEQ ID NO: 3 (the full-length polypeptide sequence of human IL-4):
SEQ ID NO: 4 (the full-length polypeptide sequence of mouse IL-4):

### Immunosuppressive Cells

Within scope of this disclosure are immunosuppressive cells or suppressor cells. As used herein, the term "immunosuppressive cells" or "suppressor cells" refers to a myeloid-derived cell population that inhibits or prevents the activation, or in another embodiment, the effector function, of another effector cell, such as a macrophage (in particular, IL-4Rα⁺ macrophage) by, *inter alias,* inducing inhibitory Fc receptor (e.g., FcyRIIB). The immunosuppressive or suppressor cells can be a homogenous population or a heterogeneous population.

Effector cells are leukocytes which express one or more FcRs and perform effector functions. Preferably, the cells express at least one type of an activating Fc receptor (such as, FcyRIII) and perform ADCC effector function. Examples of human leukocytes which mediate ADCC include peripheral blood mononuclear cells (PBMC), natural killer (NK) cells, monocytes, and neutrophils, with PBMCs cells being preferred. The effector cells may be isolated from a native source thereof, e.g., from blood or PBMCs as described herein. As mentioned above, the effector cells are preferably IL-4Rα⁺ macrophages.

Immunosuppressive or suppressor cells of this disclosure can be prepared by methods disclosed herein. In one example, the immunosuppressive or suppressor cells are macrophages or dendritic cells that have been treated with sFc. To that end, one can contact a plurality of myeloid cells from a donor mammal with a polypeptide containing a Fc region that has a N-linked biantennary oligosaccharide having a terminal sialic acid connected to galactose by an α 2,6 linkage for a period of time; and then isolate or enrich macrophages or dendritic cells from the plurality of cells to obtain immunosuppressive cells. Once administered to a recipient mammal, the immunosuppressive cells up-regulate expression of IL-33 in the recipient mammal. In another example, the immunosuppressive or suppressor cells are basophils. One can prepare such cells by contacting a plurality of myeloid cells from a donor mammal with an agonist of IL-33 receptor for a period of time; and, isolating or enriching basophils from the plurality of cells to obtain the immunosuppressive cells.

As used herein, the term "contacting" a target cell or cell population refers to both direct and indirect exposure of cell or cell population to an indicated agent or item. In one embodiment, contact of cells to a peptide, protein, cytokine, growth factor, dendritic cell, or combination thereof, is direct or indirect. In one example, contacting a cell may comprise direct injection of the cell through any means well known in the art, such as microinjection. It is also envisaged, in another example, that contacting or supplying to the cell is indirect, such as via provision in a culture medium that surrounds the cell, or administration to a subject, via any route well known in the art, and as described herein.

An "agonist" is a compound that interacts with a target to cause or promote an increase in the activation of the target. An agonist of IL-33 receptor refers to an agent that binds to an IL-33 receptor and triggers a cellular response mediated by the IL-33 receptor. Examples of an IL-33-receptor agonist include, but are not limited to, small molecule IL-33-receptor agonists, IL-33-receptor activating antibodies, as well as homologs or orthologs of IL-33 ligand (e.g., SEQ ID NOs: 1 and 2), which mimics the action of a naturally occurring IL-33.

IL-33 or Interleukin 33 is a cytokine belonging to the IL-1 superfamily. As disclosed herein, IL-33 triggers IL-4 anti-inflammatory activity. More specifically, sFc administration results in the production of IL-33, which, in turn, induces expansion of IL-4 producing basophils that promote increased expression of the inhibitory Fc receptor, FcyRIIB, on effector macrophages. Systemic administration of IL-33 or IL-4 upregulates FcyRIIB on macrophages, and suppresses serum-induced arthritis, while α-IL-33Rα treatment obscured IVIG protection of K/BxN inflammation. Thus, IL-33-treated/primed basophils are effective at suppressing arthritic inflammation, reducing serum IL-6 levels, and curbing leukocyte infiltration to arthritic paws.

As disclosed herein, besides IL-33, IL-4 is also involved in the above-mentioned DC-SIGN-Th2 pathway initiated by IVIG/sFc for suppressing unwanted immune responses. IL-4 or Interleukin-4 is a cytokine that induces differentiation of naive helper T cells (Th0 cells) to Th2 cells. The Th2 cytokine IL-4 has been shown to upregulate FcyRIIB surface expression on peripheral monocytes (Pricop et al. J Immunol 166, 531-537 (2001)), and increase the threshold for activation by pathogenic immune complexes, consistent with the FcyRIIB requirement of IVIG (Nimmerjahn et al. Annu Rev Immunol 26, 513-533 (2008); Bruhns et al. Immunity 18, 573-581 (2003); and Samuelsson et al. Science 291, 484-486 (2001)). As disclosed in the examples below, IVIG's anti-inflammatory activity requires IL-4 signaling.

While various IL-4 or IL-33 preparations can be used to practice the methods disclosed herein, highly purified or isolated IL-4 or IL-33 is preferred. Examples include human or mouse IL-4 or IL-33 (SEQ ID NOs: 1-4 shown above) and other variants having substantially the same biological activity as that having the sequence of any one of SEQ ID NOs: 1-4.

An "isolated" polypeptide or protein refers to a polypeptide or protein that has been separated from other proteins, lipids, and nucleic acids with which it is naturally associated. The polypeptide/protein can constitute at least 10% (i.e., any percentage between 10% and 100%, e.g., 20%, 30%, 40%, 50%, 60%, 70 %, 80%, 85%, 90%, 95%, and 99%) by dry weight of the purified preparation. Purity can be measured by any appropriate standard method, for example, by column chromatography, polyacrylamide gel electrophoresis, or HPLC analysis. An isolated polypeptide/protein described in the invention can be purified from a natural source, produced by recombinant DNA techniques, or by chemical methods. A functional equivalent of IL-4 or IL-33 refers to a subset of agonists of IL-4 receptor or IL-33 receptor, i.e., a polypeptide derivative of the IL-4 or IL-33 polypeptide, e.g., a protein having one or more point mutations, insertions, deletions, truncations, a fusion protein, or a combination thereof. It retains substantially the activity of the IL-4 or IL-33 polypeptide, i.e., the ability to bind to the respective receptor and trigger the respective cellular response. The isolated polypeptide can contain SEQ ID NO: 1, 2, 3, or 4, or a functional fragment thereof. In general, the functional equivalent is at least 75% (e.g., any number between 75% and 100%, inclusive, e.g., 70 %, 80%, 85%, 90%, 95%, and 99%) identical to SEQ ID NO: 1, 2, 3, or 4.

The amino acid composition of the IL-4 or IL-33 polypeptide described herein may vary without disrupting the ability of the polypeptide to bind to the respective receptor and trigger the respective cellular response. For example, it can contain one or more conservative amino acid substitutions. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a predicted nonessential amino acid residue in SEQ ID NO: 1, 2, 3, or 4 is preferably replaced with another amino acid residue from the same side chain family. Alternatively, mutations can be introduced randomly along all or part of the sequences, such as by saturation mutagenesis, and the resultant mutants can be screened for the ability to bind to the respective receptor and trigger the respective cellular response to identify mutants that retain the activity as descried below in the examples.

An IL-4 or IL-33 polypeptide as described herein can be obtained as a naturally occurring polypeptide or a recombinant polypeptide. To prepare a recombinant polypeptide, a nucleic acid encoding it (e.g., SEQ ID NO: 1, 2, 3, or 4) can be linked to another nucleic acid encoding a fusion partner, e.g., glutathione-s-transferase (GST), 6x-His epitope tag, or M13 Gene 3 protein. The resultant fusion nucleic acid expresses in suitable host cells a fusion protein that can be isolated by methods known in the art. The isolated fusion protein can be further treated, e.g., by enzymatic digestion, to remove the fusion partner and obtain the recombinant polypeptide of this invention.

All of naturally occurring IL-4 or IL-33, genetic engineered IL-4 or IL-33, and chemically synthesized IL-4 or IL-33 can be used to practice the invention disclosed therein. IL-4 or IL-33 obtained by recombinant DNA technology may have the same amino acid sequence as naturally occurring IL-4 or IL-33 (SEQ ID NO: 1, 2 ,3, or 4) or an functionally equivalent thereof. The term "IL-4" or "IL-33" also covers chemically modified IL-4 or IL-33. Examples of chemically modified IL-4 or IL-33 include IL-4 or IL-33 subjected to conformational change, addition or deletion of a sugar chain, and IL-4 or IL-33 to which a compound such as polyethylene glycol has been bound. Once purified and tested by standard methods or according to the methods described in the examples below, IL-4 or IL-33 can be included in pharmaceutical composition.

### Identification of Compounds for Treating Inflammatory Disorders

The present disclosure relates in part to identifying modulators of immune response. This can be carried out by identifying modulators of the expression of IL-33. For example, activators of IL-33 expression can mediate the DC-SIGN-Th2 pathway disclosed herein to promote increased *in vivo* expansion of IL-4⁺ basophils or expression of the FcyRIIB receptor on effector macrophages, thereby repressing inflammatory immune response. Accordingly, the activators can be used for treating inflammatory disorders.

The methods may entail any assay available to the artisan, from screening of large libraries of candidate test compounds, to assays which may focus on a related subset or class of compounds (such as antibodies or related Fc fragments), to assays focusing on specific structural attributes which may provide for selection of an enhanced Fc antibody fragment (such as an α2,6 sialylated Fc fragment) more likely to modulate the expression of IL-33.

Test compounds to be screened (e.g., proteins, peptides, peptidomimetics, peptoids, antibodies, small molecules, or other drugs) can be obtained using any of the numerous approaches in combinatorial library methods known in the art. Such libraries include: peptide libraries, peptoid libraries (libraries of molecules having the functionalities of peptides, but with a novel, non-peptide backbone that is resistant to enzymatic degradation); spatially addressable parallel solid phase or solution phase libraries; synthetic libraries obtained by deconvolution or affinity chromatography selection; and the "one-bead one-compound" libraries. See, e.g., Zuckermann et al. 1994, J. Med. Chem. 37:2678-2685; and Lam, 1997, Anticancer Drug Des. 12:145. Examples of methods for the synthesis of molecular libraries can be found in, e.g., DeWitt et al., 1993, PNAS USA 90:6909; Erb et al., 1994, PNAS USA 91:11422; Zuckermann et al., 1994, J. Med. Chem. 37:2678; Cho et al., 1993, Science 261:1303; Carrell et al., 1994, Angew. Chem. Int. Ed. Engl. 33:2059; Carell et al., 1994, Angew. Chem. Int. Ed. Engl. 33:2061; and Gallop et al., 1994 J. Med. Chem. 37:1233. Libraries of compounds may be presented in solution (e.g., Houghten, 1992, Biotechniques 13:412-421), or on beads (Lam, 1991, Nature 354:82-84), chips (Fodor, 1993, Nature 364:555-556), bacteria (U.S. Patent No. 5,223,409), spores (U.S. Patent No. 5,223,409), plasmids (Cull et al., 1992, PNAS USA 89:1865-1869), or phages (Scott and Smith 1990, Science 249:386-390; Devlin, 1990, Science 249:404-406; Cwirla et al., 1990, PNAS USA 87:6378-6382; Felici 1991, J. Mol. Biol. 222:301-310; and U.S. Patent No. 5,223,409).

Within scope of the present disclosure are methods of screening for compounds which (i) modulate (e.g., stimulate) activity of the DC-SIGN receptor type so as to promote an increase in expression of IL-33 which may affect an expansion of IL-4⁺ basophils and/or an increase in expression of the FcyRIIB receptor in a secondary macrophage; or (ii) increase the expression of DNA or RNA encoding an IL-33 protein (iii) stimulate a reporter gene linked to an IL-33 promoter responsive to downstream signaling pathway initiated by α2,6 sialylated Fc binding to a DC-SIGN receptor type.

Compounds that modulate the expression of DNA or RNA encoding IL-33 may be detected by a variety of assays. The assay may be a simple "yes/no" assay to determine whether there is a change in expression. The assay may be made quantitative by comparing the expression in a test sample with the levels of expression in a standard sample. The various assays which may be utilized to identify compounds which modulate the expression of IL-33 also include but are not limited to assays conducted in cell free systems, in one or more isolated cell types, in organisms (such as transgenic animals), or a combination thereof. Such assays may identify a developmental candidate compound which increases the expression of IL-33 so as to affect an expansion of IL-4⁺ basophils and/or an increase in expression of the FcγRIIB receptor in a secondary effector cell. A modulator may be a compound which alters the transcription of the IL-33 gene, translation of the IL-33 protein, or stability of the IL-33 protein.

Any polynucleotide sequence that encodes a functional IL-33 expression element so as to affect proper expression of IL-33 may be utilized in the assays discussed herein. An IL-33 expression element refers to a polynucleotide sequence containing a regulatory sequence that is inducible by, or otherwise responsive to, a regulator of IL-33, such as sFc or IVIG disclosed herein. The term "regulatory sequence" includes promoters, enhancers and other expression control elements (e.g., polyadenylation signals). As examples, a polynucleotide which may be utilized in constructing an appropriate DNA expression vector is a DNA molecule having an open reading frame encoding a reporter gene that is operably linked to an IL-33 promoter.

Any such polynucleotide as mentioned above or a biologically equivalent polynucleotide available to the artisan for the same intended purpose may be inserted into an appropriate expression vector and linked with other DNA molecules, i.e., DNA molecules to which the IL-33 gene are not naturally linked, to form "recombinant DNA molecules" expressing this receptor. These vectors may be comprised of DNA or RNA; for most cloning purposes DNA vectors are preferred. Typical vectors include plasmids, modified viruses, bacteriophage and cosmids, yeast artificial chromosomes and other forms of episomal or integrated DNA. It is well within the purview of the artisan to determine an appropriate vector for a particular use.

A variety of mammalian expression vectors may be used to express the above-mentioned IL-33 expression element in mammalian cells. As noted above, expression vectors are defined herein as DNA sequences that are required for the transcription of cloned DNA and the translation of their mRNAs in an appropriate host. Such vectors can be used to express eukaryotic DNA in a variety of hosts such as bacteria, blue green algae, plant cells, insect cells and animal cells. Specifically designed vectors allow the shuttling of DNA between hosts such as bacteria-yeast or bacteria-animal cells. An appropriately constructed expression vector should contain: an origin of replication for autonomous replication in host cells, selectable markers, a limited number of useful restriction enzyme sites, a potential for high copy number, and active promoters. A promoter is defined as a DNA sequence that directs RNA polymerase to bind to DNA and initiate RNA synthesis. A strong promoter is one which causes mRNAs to be initiated at high frequency. Expression vectors may include, but are not limited to, cloning vectors, modified cloning vectors, specifically designed plasmids or viruses. Commercially available mammalian expression vectors which may be suitable, include but are not limited to, pcDNA3.neo (Invitrogen), pcDNA3.1 (Invitrogen), pCI-neo (Promega), pLITMUS28, pLITMUS29, pLITMUS38 and pLITMUS39 (New England Bioloabs), pcDNAI, pcDNAIamp (Invitrogen), pcDNA3 (Invitrogen), pMClneo (Stratagene), pXT1 (Stratagene), pSG5 (Stratagene), EBO-pSV2-neo (ATCC 37593) pBPV-1(8-2) (ATCC 37110), pdBPV-MMTneo(342-12) (ATCC 37224), pRSVgpt (ATCC 37199), pRSVneo (ATCC 37198), pSV2-dhfr (ATCC 37146), pUCTag (ATCC 37460), and IZD35 (ATCC 37565).

In one example, the above described assays may also be based on measurement of induction and expression of a reporter gene or epitope tag within a recombinant DC-SIGN⁽⁺⁾ cell. The art is now replete with various reporter genes and epitope tag polypeptides available to the artisan that will be suitable to measuring the ability of a test compound to modulate expression of a gene, such as IL-33. The artisan will be capable of mixing and matching these various research tools without undue experimentation. For example, various reporter genes include but are not limited to green fluorescent protein ("GFP") or functional protein/polypeptide derivatives thereof. GFP genes and various mutants (which may fluoresce at different wavelengths and improved spectral properties) have been identified in a variety of organisms in the phyla hydrozoa, cnidaria, anthozoa and ctenophora. Select GFP variants include blue fluorescent protein ("BPF"), yellow fluorescent protein (YFP), and cyan fluorescent protein (CFP). For additional suitable fluorescent proteins, see Matz et al., 1999, Nature Biotechnology 17:969-973. Other suitable reporter genes include chloramphenicol acetyl transferase ("CAT") and other enzyme detection systems, such as beta-galactosidase (β-gal"); firefly luciferase, bacterial luciferase, or secreted alkaline phosphate ("SEAP"). Other examples of suitable reporter genes include those which encode proteins conferring drug/antibiotic resistance to the host mammalian cell. The amount of transcription from the reporter gene may be measured using any suitable method known in the art, including detecting RNA expression via Northern blots, protein expression by any detection method known to that protein, such as a characteristic stain or an intrinsic activity (e.g., such as enzyme activity, or giving rise to a detection signal based on fluorescence, color, or luminescence, as discussed above). It is also possible that the activated reporter gene will provide an expressed protein which provides a growth advantage for the cell (e.g., be enhancing cell viability, relieving a cell nutritional requirement, and/or providing drug resistance). Other reporter genes may encode cell surface proteins for which antibodies or ligands are available. Expression of the reporter gene allows cells to be detected or affinity purified by the presence of the surface protein. Alternatively, the fused polypeptide is an epitope tag, examples of which include but are not limited to a Myc tag, a Flag tag, a His tag, a Leucine tag, an IgG tag, a biotinylation sequence site ("BSS," i.e., a streptavidin tag) and the like.

Compounds identified by the method described above are candidates useful for treating inflammatory disorders. One can further verify the efficacy of a compound thus-identified using an animal model, such as a transgenic mouse, as described below. Any statistically significant increase in *in vivo* expansion of IL-4⁺ basophils or expression of the FcyRIIB receptor on effector macrophages indicates the compound is a candidate for treating the disorders mentioned below.

### Evaluating of DC-SIGN Modulating Compositions

As disclosed herein, a DC-SIGN receptor type interacts with IgG antibodies or Fc fragments to promote an anti-inflammatory effect associated with known IVIG treatment protocols. Compounds that modulate (and preferably act as an agonist) of a DC-SIGN receptor type are useful in regulating such anti-inflammatory response. A modulator of particular interest is a compound which acts as an agonist to the DC-SIGN receptor type. Such a compound will show the ability to mediate a signal from a DC-SIGN⁺ cell (such as a dendritic cell) to an effector macrophage, causing an increase in expression of the FcγRIIB receptor, which in turn inhibits the cellular-mediated inflammatory response normally generated from these macrophages in response to relevant autoantibodies.

Having obtained compositions having a DC-SIGN modulating compound, it is desirable to be able to compare the DC-SIGN modulating activity (i.e., potency) of these DC-SIGN modulating compositions to that of known standards. Such "known standards" are established by quantifying the characteristics of a DC-SIGN modulating composition of known therapeutic efficacy, e.g., IVIG. Suitable characteristics for analysis include: the amount of DC-SIGN binding activity (i.e., the amount of DC-SIGN binding compound in the composition); the amount of DC-SIGN modulating activity (e.g., the efficacy of the DC-SIGN binding composition in a cell based assay of DC-SIGN modulation); and, the anti-inflammatory activity of a DC-SIGN-modulating composition in an *in vivo* assay. See U.S. Patent No. 7846744. Such comparisons with a DC-SIGN modulating composition of known therapeutic efficacy are useful for, e.g., standardization of the therapeutic dose of DC-SIGN modulating compositions, or providing a functional comparison of the DC-SIGN modulating activity of biosimilars. Accordingly, the disclosure also provides methods for comparing the characteristics of a DC-SIGN modulating composition to those of a known standard.

The present invention provides an *in vivo* method of determining the anti-inflammatory activity of a DC-SIGN-modulating composition comprising a polypeptide containing a Fc region that has a N-linked biantennary oligosaccharide having a terminal sialic acid connected to galactose by an α 2,6 linkage, comprising (a) administering a DC-SIGN-binding composition comprising a polypeptide containing a Fc region that has a N-linked biantennary oligosaccharide having a terminal sialic acid connected to galactose by an α 2,6 linkage to a mouse; and (b) measuring the expression level of IL-33 mRNA in splenocytes of the mouse, wherein an increase in the expression level of IL-33 mRNA in splenocytes of the mouse is indicative of the anti-inflammatory activity of the DC-SIGN-binding composition. The expression level of IL-33 mRNA can be obtained by techniques known in the art, e.g., qPCR.

Any art recognized mouse model of antibody mediated inflammation can be used for this method, including, but not limited to, those disclosed herein. Any modified mouse including, but not limited to, a mouse expressing a human DC-SIGN receptor type or lectin binding domain thereof may be used.

In the above described assays, the presence, level, or absence of the polypeptide or nucleic acid in a test sample can be evaluated by obtaining a test sample from a test subject and contacting the test sample with a compound or an agent capable of detecting the polypeptide or nucleic acid (e.g., mRNA probe, genomic cDNA probe, or cDNA probe). The "test sample" can include tissues, cells and biological fluids isolated from a subject, as well as tissues, cells and fluids present within a subject. The level of expression of the gene can be measured in a number of ways, including, but not limited to, measuring the mRNA encoded by the gene; measuring the amount of polypeptide encoded by the gene; or measuring the activity of polypeptide encoded by the gene.

The level of mRNA corresponding to the gene in a cell can be determined both by *in situ* and by *in vitro* formats. Messenger RNA isolated from a test sample can be used in hybridization or amplification assays that include, but are not limited to, Southern or Northern analyses, PCR analyses, and probe arrays. For example, one method for the detection of mRNA levels involves contacting the isolated mRNA with a nucleic acid probe that can hybridize to the mRNA encoded by the gene. The probe can be a full-length nucleic acid, or a portion thereof, such as an oligonucleotide of at least 10 nucleotides in length and sufficient to specifically hybridize under stringent conditions to mRNA or genomic DNA.

In one format, mRNA (or cDNA prepared from it) is immobilized on a surface and contacted with the probes, for example, by running the isolated mRNA on an agarose gel and transferring the mRNA from the gel to a membrane, such as nitrocellulose. In another format, the probes are immobilized on a surface and the mRNA (or cDNA) is contacted with the probes, for example, in a gene chip array. A skilled artisan can adapt known mRNA detection methods for detecting the level of mRNA.

The level of mRNA (or cDNA prepared from it) in a sample encoded by one or more of the above-mentioned genes can be evaluated with nucleic acid amplification, e.g., by standard PCR (U.S. Patent No. 4,683,202), RT-PCR (Bustin S. J Mol Endocrinol. 25:169-93, 2000), quantitative PCR (Ong Y. et al., Hematology. 7:59-67, 2002), real time PCR (Ginzinger D. Exp Hematol. 30:503-12, 2002), and *in situ* PCR (Thaker V. Methods Mol Biol. 115:379-402, 1999), or any other nucleic acid amplification method, followed by the detection of the amplified molecules using techniques known in the art. As used herein, "amplification primers" are defined as being a pair of nucleic acid molecules that can anneal to 5' or 3' regions of a gene (plus and minus strands, respectively, or vice-versa) and contain a short region in between. Under appropriate conditions and with appropriate reagents, such primers permit the amplification of a nucleic acid molecule having the nucleotide sequence flanked by the primers.

For *in situ* methods, a cell or tissue sample can be prepared and immobilized on a support, such as, but not limited to, a glass slide, and then contacted with a probe that can hybridize to genomic DNA on chromosomes or mRNA that encodes the corresponding polypeptide.

In another embodiment, the methods of the described invention further include contacting a control sample with a compound or agent capable of detecting mRNA, or genomic DNA, and comparing the presence of mRNA or genomic DNA in the control sample with the presence of mRNA or genomic DNA in the test sample.

A variety of methods can be used to determine the level of one or more of the above-mentioned polypeptide. In general, these methods include contacting an agent that selectively binds to the polypeptide, such as an antibody, to evaluate the level of polypeptide in a sample. Antibodies can be polyclonal, or monoclonal. An intact antibody, or a fragment thereof (e.g., Fab or F(ab')₂) also can be used. In another embodiment, the antibody bears a detectable label. The term "labeled", with regard to the probe or antibody, is intended to encompass direct labeling of the probe or antibody by physically linking a detectable substance to the probe or antibody, as well as indirect labeling of the probe or antibody by reactivity with a detectable substance. For example, an antibody with a rabbit Fc region can be indirectly labeled using a second antibody directed against the rabbit Fc region, wherein the second antibody is coupled to a detectable substance. Examples of detectable substances are provided herein. Appropriate detectable substance or labels include, but are not limited to, radio isotopes (for example, but not limited to, ¹²⁵I, ¹³¹I, ³⁵S, ³H, or ³²P), enzymes (for example, but not limited to, alkaline phosphatase, horseradish peroxidase, luciferase, or β-glactosidase), fluorescent moieties or proteins (for example, but not limited to, fluorescein, rhodamine, phycoerythrin, GFP, or BFP), or luminescent moieties (for example, but not limited to, Qdot™ nanoparticles by the Quantum Dot Corporation, Palo Alto, CA).

The detection methods can be used to detect one or more of the above-mentioned polypeptide in a biological sample *in vitro* as well as *in vivo. In vitro* techniques for detection of the polypeptide include ELISAs, immuno-precipitations, immunofluorescence, EIA, RIA, and Western blotting analysis. *In vivo* techniques for detection of the polypeptide include introducing into a subject a labeled antibody. For example, the antibody can be labeled with a detectable substance as described above. The presence and location of the detectable substance in a subject can be detected by standard imaging techniques.

As used herein, "antibody" is used in the broadest sense and specifically covers monoclonal antibodies (including full length monoclonal antibodies), polyclonal antibodies, multispecific antibodies (e.g., bispecific antibodies), and antibody fragments so long as they exhibit the desired biological activity.

As used herein, "antibody fragments", may comprise a portion of an intact antibody, generally including the antigen binding or variable region of the intact antibody or the Fc region of an antibody which retains FcR binding capability. Examples of antibody fragments include linear antibodies; single-chain antibody molecules; and multispecific antibodies formed from antibody fragments. The antibody fragments preferably retain at least part of the hinge and optionally the CH1 region of an IgG heavy chain. More preferably, the antibody fragments retain the entire constant region of an IgG heavy chain, and include an IgG light chain.

As used herein, the term "Fc fragment" or "Fc region" is used to define a C-terminal region of an immunoglobulin heavy chain. The "Fc region" may be a native sequence Fc region or a variant Fc region. Although the boundaries of the Fc region of an immunoglobulin heavy chain might vary, the human IgG heavy chain Fc region is usually defined to stretch from an amino acid residue at position Cys226, or from Pro230, to the carboxyl-terminus thereof.

A "native sequence Fc region" comprises an amino acid sequence identical to the amino acid sequence of an Fc region found in nature. A "variant Fc region" as appreciated by one of ordinary skill in the art comprises an amino acid sequence which differs from that of a native sequence Fc region by virtue of at least one "amino acid modification." Preferably, the variant Fc region has at least one amino acid substitution compared to a native sequence Fc region or to the Fc region of a parent polypeptide, e.g., from about one to about ten amino acid substitutions, and preferably from about one to about five amino acid substitutions in a native sequence Fc region or in the Fc region of the parent polypeptide. The variant Fc region herein will preferably possess at least about 80% homology with a native sequence Fc region and/or with an Fc region of a parent polypeptide, and more preferably at least about 90% homology therewith, more preferably at least about 95% homology therewith, even more preferably, at least about 99% homology therewith.

The terms "Fc receptor" or "FcR" are used to describe a receptor that binds to the Fc region of an antibody. In one embodiment of the invention, FcR is a native sequence human FcR. In another embodiment, FcR, including human FcR, binds an IgG antibody (a gamma receptor) and includes receptors of the FcγRI, FcγRII, and FcγRIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain (see review in Daron, Annu Rev Immunol, 15, 203-234 (1997); FcRs are reviewed in Ravetch and Kinet, Annu Rev Immunol, 9, 457-92 (1991); Capel et al., Immunomethods, 4, 25-34 (1994); and de Haas et al., J Lab Clin Med, 126, 330-41 (1995), Nimmerjahn and Ravetch 2006, Ravetch Fc Receptors in Fundemental Immunology, ed William Paul 5th Ed.).

### Compositions

Within the scope of this disclosure is a composition that contains a suitable carrier and one or more of the agents described above, such as bone marrow-derived sFc-treated hDC-SIGN⁺ macrophages or dendritic cells, IL-4, IL-33, IL-33-treated basophils, or agents identified by screening methods disclosed above. The composition can be a pharmaceutical composition that contains a pharmaceutically acceptable carrier or a cosmetic composition that contains a cosmetically acceptable carrier.

The term "pharmaceutical composition" refers to the combination of an active agent with a carrier, inert or active, making the composition especially suitable for diagnostic or therapeutic use *in vivo* or *ex vivo.* A "pharmaceutically acceptable carrier," after administered to or upon a subject, does not cause undesirable physiological effects. The carrier in the pharmaceutical composition must be "acceptable" also in the sense that it is compatible with the active ingredient and can be capable of stabilizing it. One or more solubilizing agents can be utilized as pharmaceutical carriers for delivery of an active compound. Examples of a pharmaceutically acceptable carrier include, but are not limited to, biocompatible vehicles, adjuvants, additives, and diluents to achieve a composition usable as a dosage form. Examples of other carriers include colloidal silicon oxide, magnesium stearate, cellulose, and sodium lauryl sulfate.

The above-described composition, in any of the forms described above, can be used for treating disorders characterized by inflammation. An effective amount refers to the amount of an active compound/agent that is required to confer a therapeutic effect on a treated subject. Effective doses will vary, as recognized by those skilled in the art, depending on the types of diseases treated, route of administration, excipient usage, and the possibility of co-usage with other therapeutic treatment.

A pharmaceutical composition of this disclosure can be administered parenterally, orally, nasally, rectally, topically, or buccally. The term "parenteral" as used herein refers to subcutaneous, intracutaneous, intravenous, intrmuscular, intraarticular, intraarterial, intrasynovial, intrasternal, intrathecal, intralesional, or intracranial injection, as well as any suitable infusion technique.

A sterile injectable composition can be a solution or suspension in a non-toxic parenterally acceptable diluent or solvent. Such solutions include, but are not limited to, 1,3-butanediol, mannitol, water, Ringer's solution, and isotonic sodium chloride solution. In addition, fixed oils are conventionally employed as a solvent or suspending medium (e.g., synthetic mono- or diglycerides). Fatty acid, such as, but not limited to, oleic acid and its glyceride derivatives, are useful in the preparation of injectables, as are natural pharmaceutically acceptable oils, such as, but not limited to, olive oil or castor oil, polyoxyethylated versions thereof. These oil solutions or suspensions also can contain a long chain alcohol diluent or dispersant such as, but not limited to, carboxymethyl cellulose, or similar dispersing agents. Other commonly used surfactants, such as, but not limited to, Tweens or Spans or other similar emulsifying agents or bioavailability enhancers, which are commonly used in the manufacture of pharmaceutically acceptable solid, liquid, or other dosage forms also can be used for the purpose of formulation.

A composition for oral administration can be any orally acceptable dosage form including capsules, tablets, emulsions and aqueous suspensions, dispersions, and solutions. In the case of tablets, commonly used carriers include, but are not limited to, lactose and corn starch. Lubricating agents, such as, but not limited to, magnesium stearate, also are typically added. For oral administration in a capsule form, useful diluents include, but are not limited to, lactose and dried corn starch. When aqueous suspensions or emulsions are administered orally, the active ingredient can be suspended or dissolved in an oily phase combined with emulsifying or suspending agents. If desired, certain sweetening, flavoring, or coloring agents can be added.

Pharmaceutical compositions for topical administration as described herein can be formulated as solutions, ointments, creams, suspensions, lotions, powders, pastes, gels, sprays, aerosols, or oils. Alternatively, topical formulations can be in the form of patches or dressings impregnated with active ingredient(s), which can optionally comprise one or more excipients or diluents. In some preferred examples, the topical formulations include a material that would enhance absorption or penetration of the active agent(s) through the skin or other affected areas. The topical composition is useful for treating inflammatory disorders in the skin, including, but not limited to eczema, acne, rosacea, psoriasis, contact dermatitis, and reactions to poison ivy.

A topical composition contains a safe and effective amount of a dermatologically acceptable carrier suitable for application to the skin. A "cosmetically acceptable" or "dermatologically-acceptable" composition or component refers a composition or component that is suitable for use in contact with human skin without undue toxicity, incompatibility, instability, allergic response, and the like. The carrier enables an active agent and optional component to be delivered to the skin at an appropriate concentration(s). The carrier thus can act as a diluent, dispersant, solvent, or the like to ensure that the active materials are applied to and distributed evenly over the selected target at an appropriate concentration. The carrier can be solid, semi-solid, or liquid. The carrier can be in the form of a lotion, a cream, or a gel, in particular one that has a sufficient thickness or yield point to prevent the active materials from sedimenting. The carrier can be inert or possess dermatological benefits. It also should be physically and chemically compatible with the active components described herein, and should not unduly impair stability, efficacy, or other use benefits associated with the composition. The topical composition may be a cosmetic or dermatologic product in the form known in the art for topical or transdermal applications, including solutions, aerosols, creams, gels, patches, ointment, lotion, or foam.

### Treatment Methods

Disclosed are methods for treating in a subject an inflammatory disorder. The term "inflammatory disorder" refers to a disorder that is characterized by abnormal or unwanted inflammation, such as an autoimmune disease. Autoimmune diseases are disorders characterized by the chronic activation of immune cells under non-activating conditions. Examples include psoriasis, inflammatory bowel diseases (e.g., Crohn's disease and ulcerative colitis), rheumatoid arthritis, psoriatic arthritis, multiple sclerosis, lupus, type I diabetes, primary biliary cirrhosis, and transplant.

Other examples of inflammatory disorders that can be treated by the methods of this disclosure include asthma, myocardial infarction, stroke, inflammatory dermatoses (e.g., dermatitis, eczema, atopic dermatitis, allergic contact dermatitis, urticaria, necrotizing vasculitis, cutaneous vasculitis, hypersensitivity vasculitis, eosinophilic myositis, polymyositis, dermatomyositis, and eosinophilic fasciitis), acute respiratory distress syndrome, fulminant hepatitis, hypersensitivity lung diseases (e.g., hypersensitivity pneumonitis, eosinophilic pneumonia, delayed-type hypersensitivity, interstitial lung disease (ILD), idiopathic pulmonary fibrosis, and ILD associated with rheumatoid arthritis), and allergic rhinitis. Additional examples also include myasthenia gravis, juvenile onset diabetes, glomerulonephritis, autoimmune throiditis, ankylosing spondylitis, systemic sclerosis, acute and chronic inflammatory diseases (e.g., systemic anaphylaxia or hypersensitivity responses, drug allergies, insect sting allergies, allograft rejection, and graft-versus-host disease), and Sjogren's syndrome.

A "subject" refers to a human and a non-human animal. Examples of a non-human animal include all vertebrates, e.g., mammals, such as non-human mammals, non-human primates (particularly higher primates), dog, rodent (e.g., mouse or rat), guinea pig, cat, and rabbit, and non-mammals, such as birds, amphibians, reptiles, etc. In one example, the subject is a human. In another example, the subject is an experimental, non-human animal or animal suitable as a disease model.

A subject to be treated for an inflammatory disorder can be identified by standard diagnosing techniques for the disorder. Optionally, the subject can be examined for the level or percentage of one or more of the above-mentioned cytokines or cells a test sample obtained from the subject by methods described below. If the binding level or percentage is at or below a threshold value (which can be obtained from a normal subject), the subject is a candidate for treatment described herein. To confirm the inhibition or treatment, one can evaluate and/or verify the level or percentage of one or more of the above-mentioned cytokines or cells in the subject after treatment.

"Treating" or "treatment" refers to administration of a compound or agent to a subject who has a disorder with the purpose to cure, alleviate, relieve, remedy, delay the onset of, prevent, or ameliorate the disorder, the symptom of the disorder, the disease state secondary to the disorder, or the predisposition toward the disorder.

An "effective amount" or "therapeutically effective amount" refers to an amount of the compound or agent that is capable of producing a medically desirable result in a treated subject. The treatment method can be performed *in vivo* or *ex vivo,* alone or in conjunction with other drugs or therapy. A therapeutically effective amount can be administered in one or more administrations, applications or dosages and is not intended to be limited to a particular formulation or administration route.

The agent can be administered *in vivo* or *ex vivo,* alone or co-administered in conjunction with other drugs or therapy, i.e., a cocktail therapy. As used herein, the term "co-administration" or "co-administered" refers to the administration of at least two agents or therapies to a subject. In some examples, the co-administration of two or more agents/therapies is concurrent. In other examples, a first agent/therapy is administered prior to a second agent/therapy. Those of skill in the art understand that the formulations and/or routes of administration of the various agents/therapies used may vary.

In an *in vivo* approach, a compound or agent is administered to a subject. Generally, the compound or agent is suspended in a pharmaceutically-acceptable carrier (such as, for example, but not limited to, physiological saline) and administered orally or by intravenous infusion, or injected or implanted subcutaneously, intramuscularly, intrathecally, intraperitoneally, intrarectally, intravaginally, intranasally, intragastrically, intratracheally, or intrapulmonarily.

The dosage required depends on the choice of the route of administration; the nature of the formulation; the nature of the patient's illness; the subject's size, weight, surface area, age, and sex; other drugs being administered; and the judgment of the attending physician. Suitable dosages are in the range of 0.01-100 mg/kg or 0.1x10⁶ to 100x10⁶ cells/dose (e.g., 10x10⁶ to 20x10⁶ cells/dose). Variations in the needed dosage are to be expected in view of the variety of compounds/agents available and the different efficiencies of various routes of administration. For example, oral administration would be expected to require higher dosages than administration by i.v. injection. Variations in these dosage levels can be adjusted using standard empirical routines for optimization as is well understood in the art. Encapsulation of the compound in a suitable delivery vehicle (e.g., polymeric microparticles or implantable devices) can increase the efficiency of delivery, particularly for oral delivery.

### Example 1: Methods and Materials

This example describes general methods and materials used in Examples 2-7.

### Mice

Eight- to twelve-week old, sex- and age-matched mice were used for all experiments in compliance with federal laws, institutional guidelines and have been approved by the Rockefeller University (New York, NY). WT C57BL/6, WT BALB/c, NOD, IL-4^{-/-}, IL-4Rα^{-/-}, Stat6^{-/-} , and IL-10^{-/-} mice were purchased from Jackson Laboratories, and maintained at the Rockefeller University animal facility. FcγRIIB^{-/-} mice were generated previously in the laboratory (Takai et al. Nature 379, 346-349 (1996)). SIGN-R1^{-/-} mice were generously provided by Dr. A. McKenzie (MRC, Cambridge, UK; see Lanoue et al. J Exp Med 200, 1383-1393 (2004)). CD11c-hDCSIGN⁺ mice were kindly provided by Dr. T. Sparwasser (Technische Universität München, Munich, Germany; see Schaefer et al. J Immunol 180, 6836-6845 (2008)). KRN TCR transgenic mice on a C57BL/6 background (K/B) were gifts from D. Mathis and C. Benoist (Harvard Medical School, Boston, MA) and were bred to NOD mice to generate K/BxN mice (Korganow et al. Immunity 10, 451-461 (1999)). K/BxN serum was prepared as described in Bruhns et al. Immunity 18, 573-581 (2003). Briefly, serum was separated from blood collected from the K/BxN mice (6-12 weeks old). Several weeks of serum collection were pooled together and frozen in aliquots to be used in the experiments described here. One intravenous injection of 200µl K/BxN serum was used to induce arthritis. Severity of arthritis was scored by clinical examination by adding the index of all four paws according to the following:
0 (unaffected),
1 (swelling of one joint),
2 (swelling of more than one joint), and
3 (severe swelling of the entire paw).
All experiments were performed at least 3 times with treatment groups of 4-5 mice, and yielded similar results.

To generate hDC-SIGN BAC transgenic mice, the BAC clone CTD2102F19 (Invitrogen) containing the hDC-SIGN gene was linearized by the Not I restriction endonuclease. The hDC-SIGN gene fragment was purified and injected into one day-old C57BL/6 embryos via pronuclear microinjection. The embryos were then implanted into ICR surrogate females and the resulting progeny were screened by PCR for the presence of the hDC9 SIGN transgene. hDC-SIGN⁺ mice were crossed to SIGN-R1^{-/-} to generate hDC-SIGN⁺/SIGN2 R1^{-/-} lines.

### Reagents and Treatments

IVIG (Octagam, Octapharma) or IVIG-derived Fcs was enriched for terminal sialic acid using SNA-agarose in the manner descried in Kaneko et al. Science 313, 670-673 (2006) (Vector Laboratories) or hypersialyated in vitro as previously described in Anthony et al. Science 320, 373-376 (2008) to generate sFc. AsialoFc was generated by digestion with Neuraminidase (NEB) pursuant to manufacturer's directions. Sialic acid enrichment and digestions were verified by lectin blotting with SNA-biotin (Vector Laboratories). IVIG and IVIG derivations were administered intravenously at 1g/kg, SNA-enriched IVIG at 0.1g/kg, and sFc at 0.03g/kg one hour prior to K/BxN sera administration.

Mice receiving cytokine:immune complexes with prolonged half-life were treated with a single intravenous (IV) injection 25µg of cytokine (IL-3, IL-4, IL-13, Peprotech, NJ) and 12.5µg of neutralizing antibody at day 0. Neutralizing antibodies used were α-IL-3 (MP2-8F8, Biolegend), α-IL4 (11B11, BD Biosciences), and α-IL-13 (eBio1316H, eBioscience). Other cytokine treatments included intraperitoneal (IP) administration 400 or 800ng of IL-25 (R&D, MN), 400ng of IL-33 (R&D, MN), or 1µg of TSLP (R&D, MN) on days 0, 1, 2, and 3. Basophils were depleted as described in Sokol et al. Nat Immunol 9, 310-318 (2008) by daily IP injection with 10µg of α-FcεRI (MAR-1, eBioscience) or hamster IgG isotype control (eBioscience) days 0-5. Alternatively, mice received a single IV injection of 30µg α-CD200RL3 in the manner described in Obata et al. Blood 110, 913-920 (2007) (Ba103, Hycult Biotech, The Netherlands) or rat IgG isotype control (BD Biosciences). IL-33Rα was blocked by IV injection of 80µg of α-IL-33Rα (DT8, MD Biosciences) or rat IgG1 isotype control (BD Biosciences) on day 0. hDC-SIGN was blocked *in vivo* by administration of 125µg E9E A8 (Cheong et al. J Immunol Methods 360, 66-75, (2010)) or isotype control mouse IgG2a (Biolegend).

Splenic RNA was purified using RNeasy Mini Kits (QIAGEN) and reverse-transcribed using Verso cDNA synthesis kit (Thermo Scientific). Quantitative RT-PCR was conducted in 7300 Real-time PCR System (Life Technologies) with specific primer-probes for mouse IL-4, IL-13, IL-33, IL-25, or rRNA (Life Technologies), respectively, and gene expression was normalized to rRNA Ct levels.

IL-6 was measured in serum by ELISA as suggested by the manufacturer (BioLegend, CA). IL-4 and IL-13 was measured in serum using an *in vivo* cytokine capture assay (IVCCA) as described in Finkelman et al. Curr Protoc Immunol Chapter 6, Unit 6 28, (2003). Briefly, biotinylated α-IL-4 antibody (clone BVD4-1D11, BioLegend) or biotinylated α-IL-13 (eBio1316HA, eBioscience) was injected and after 24h serum were collected and measured by an ELISA based assay using α-IL-4 (BVD6-24G1, BioLegend) or α-IL-13 (eBio13A, eBioscience) as capture antibodies.

Saturation binding experiments were performed as previously described in Anthony et al. Proc Natl Acad Sci U S A 6 105, 19571-19578 (2008), comparing CHO and CHO-hDC-SIGN cells or Hep-CD81 and Hep-hDC-SIGN-R cells.

### Flow Cytometry

Single-cell suspensions were prepared from peripheral blood, spleen, bone marrow, or paws from mice. After red blood cell lysis, cells were stained with the indicated monoclonal antibodies, and subjected to analysis using a FACSCalibur or LSR-II cytometer (BD Biosciences). Human leukocytes were obtained from peripheral blood (New York Blood Center) after density gradient centrifugation (Ficoll-Paque, GE Healthcare). Antibodies used for murine staining were as follows: α-CD19 (1D3), α-B220 (RA3-6B2), α-CD3ε (145-2C11), α-CD11b (M1/70), α-Ly6G (1A8),□ α-CD11c (HL3), α-I-Ab (AF6-120.1), α-hDC-SIGN/DC-SIGN-R (DCN46), α-CD49b (DX5 and HMa2), α-c-Kit (2B8), α-CD45.2 (104) from BD Biosciences, α-NKp46 (29A1.4), α-SIGN-R1 (22D1), α-CD123 (5B11) from eBioscience, α-hDC-SIGN (9E9A8), α-FceR1 (MAR-1), from Biolegend, α-FcγRIIB (K9.361) and α-hDC-SIGNR (120604) from R&D systems. Antibodies used for human cell staining were: α-CD14 (M5E2), α-CD16 (B73.1), α-CD3 (UCHT1), α-CD56 (B159), α-CD19 (SJ25C1), α-CD11c (B-Ly6), α-HLA-DR (L243 (G46-6)), α-hDC-SIGN (AZND1), from BD Biosciences, and α-hDC-SIGN (9E9A8, Biolegend). AccuCheck Counting Beads (INVITROGEN) were used to quantify cells.

### Bone Marrow Macrophage and Dendritic Cell Cultures and Transfers

Bone marrow-derived macrophages were cultured as described in Jeffrey et al. Nat Immunol 7, 274-283 (2006). Briefly, marrow was recovered from tibias and femurs from mice, and seeded in non-tissue culture treated 10-cm plates with DMEM supplemented with 10% fetal bovine serum, 2% penicillin/streptomicin (INVITROGEN), 1% glutamine 200 mM (Invitrogen), 0.1% β-mercaptoethanol, IL-3 (5ng/ml, Peprotech, NJ) and M-CSF (5ng/ml, Peprotech, NJ) in non-tissue culture treated 10cm plates overnight at 37°C, 5% CO₂. The next day, non-adherent cells were recovered, plated in 10-cm non-tissue culture treated plates in supplemented DMEM with cytokines and cultured for 5-7 days. Once the cultured cells were mature macrophages (>90% CD11b⁺ F4/80⁺ by FACS), the cells were detached and 2x10⁶ macrophages were plated per well in 6-well plates, and allowed to attach overnight. The next day the cells were pulsed with IVIG (15mg/ml), sFc (1.5mg/ml), or asialoFc (1.5mg/ml) for 30 minutes at 37°C. The cells were recovered, washed thoroughly in cold PBS, and 1x10⁶ macrophages were injected into naive recipients. One hour later, the recipient mice were treated with K/BxN sera. Dendritic cells were cultured from mouse tibia and femur bone marrow cells as described in Inaba et al. J Exp Med 176, 1693-1702 (1992). Briefly, 1x10⁶ cells/ml were plated in 24-well plates with DMEM supplemented with 10% FBS and 10ng/ml mouse granulocyte macrophage colony stimulating factor (GM-CSF, Peprotech). On day 6, loosely adherent cells were collected by gentle pipetting, and were subjected to flow cytometric analysis or bone marrow cell transfer experiments.

### Histology

Spleens or lymph nodes embedded in O.C.T. compound (Sakura Finetek) were fixed by ice-cold acetone for 10 minutes, stained with α-SIGN-R1 (22D1, eBioscience), α-hDC-SIGN or hDC-SIGN-R for 1 hour at room temperature in combination with antibodies for the specific cell populations; α-F4/80 (BM8, Invitrogen) for mouse red pulp macrophages, α-B220 for mouse B cells, α-hCD20 (2H7, Biolegend) for human B cells, and α-CD68 (Y1/82A, Biolegend) for human macrophages. Sections were visualized by wide-field fluorescence microscope (Zeiss). Human lymph node samples were from ISL Bio. Wright-Giemsa stain of sorted, cytospun basophils was performed as suggested by manufacturer (Sigma).

### Basophil Adoptive Transfers

Basophils were expanded by administering IL-3ic (Ohmori et al. J Immunol 182, 2835-2841, (2009)) as described above to WT or FcγRIIB^{-/-} mice. Five days later, mice were administered PBS or IL-33 (400ng) IP. The following day, basophils (DX5⁺ FcRI⁺ cKit⁻) were sorted using a FACSAria II (BD Biosciences). Sorted basophils were washed in cold PBS, and 0.7x10⁶ basophils were administered to naive recipient mice subsequently administered K/BxN sera.

### Example 2: Human DC-SIGN Transgenic Mice

In this example, transgenic mice having the human DC-SIGN gene were generated and examined to study roles of DC-SIGN in the context of IVIG anti-inflammatory activity.

Binding of IVIG or sFc to Specific ICAM-3 Grabbing Non-Integrin Related 1 (SIGN-R1) on splenic marginal zone macrophages suppresses autoantibody mediated inflammation (Anthony et al. Proc Natl Acad Sci U S A13 105, 19571-19578 (2008)). While the human orthologue of SIGN-R1, DC-SIGN, displayed similar binding specificity for sFc as mouse SIGN-R1, its expression pattern is broader, as it is detected systemically on myeloid derived cells, including dendritic cells, macrophages, and some monocytes (Granelli-Piperno et al. J Immunol 175, 4265-4273, (2005) and Soilleux et al. J Leukoc Biol 71, 445-457 (2002)). DC-SIGN recognizes high-mannose glycans from a variety of pathogens, and acts as a pattern recognition receptor bridging innate and adaptive immunity (Geijtenbeek et al. Nat Rev Immunol 9, 465-479 (2009)). Ligation of DC-SIGN by bacteria-derived mannosylated glycans can induce their internalization, and also synergize with other innate receptor pathways promoting inflammation and resistance to infection. In contrast, binding of sFc to DC-SIGN requires both carbohydrate and protein determinants, and results in an anti-inflammatory response (Kaneko et al. Science 313, 670-673 (2006) and Anthony et al. Proc Natl Acad Sci U S A13 105, 19571-19578 (2008)). The immunosuppressive potential of DC-SIGN has been documented following ligation by HIV8-derived gp120 or α-DC-SIGN antibody, which promotes the development of tolerogenic, IL-10 producing, dendritic cells, and interferes with TLR signaling (Gringhuis et al. Immunity 26, 605-24 616 (2007) and Hodges et al. Nat Immunol 27 8, 569-577 (2007)).

To study human DC-SIGN in the context of IVIG anti-inflammatory activity, assays were carried out to express human DC-SIGN (hDC-SIGN), driven by its endogenous promoter to reproduce the characteristically broad *in vivo* expression pattern of hDC-SIGN, in a mouse. Briefly, human BAC clones encoding the DC-SIGN gene and its regulatory regions were introduced as a transgene into mice (FIG. 1a). Once transgene mice were obtained, leukocytes or tissues from them were examined by FACS analysis or immuno-staining.

It was found that transgenic mice displayed surface expression of this human lectin on dendritic cells, macrophages, and monocytes, in the peripheral blood, bone marrow, and spleen, resembling the human expression pattern of DC-SIGN (FIGs. 6a-c), although a higher percentage of murine monocytes were found to express DC-SIGN.

### Example 3: Human DC-SIGN Conveyed Anti-Inflammatory Response of sFc

To determine if hDC-SIGN could substitute for SIGN-R1 in mediating IVIG protection, hDC-SIGN⁺ mice were crossed to SIGN-R1 deficient animals (hDC-SIGN⁺/SIGN-R1^{-/-}) and challenged with arthitogenic K/BxN serum (Korganow et al. Immunity 10, 451-461 (1999). It was found that both induction of arthritis and responsiveness to IVIG and sFc were similar in wild type (WT) mice and hDC-SIGN⁺/SIGN-R1^{-/-} animals (FIG. 1b, and FIG. 7a). In contrast, induced arthritis was not suppressed by IVIG or sFc in SIGN-R1^{-/-}mice.

These results demonstrate that hDC-SIGN expression was sufficient to trigger the IVIG and sFc anti-inflammatory response.

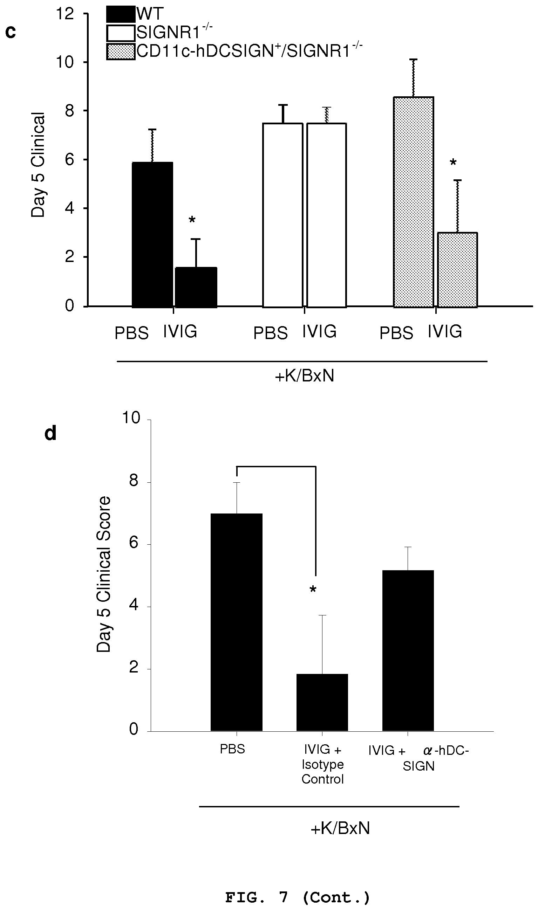

In the above-mentioned BAC clones, a related lectin, DC-SIGN-R, is linked to DC-SIGN on the BAC transgene (FIG. 1a). It was found that hDC-SIGN-R had reduced affinity to sFc as compared to hDC-SIGN (FIG. 7b). To define the contribution of hDC-SIGN-R to sFc anti-inflammatory activity, mice that expressed hDC-SIGN alone as a transgene (Schaefer et al. J Immunol 180, 6836-6845 (2008)) were crossed with SIGN-R1^{-/-} mice (CD11c-DC-SIGN/SIGN-R1^{-/-}). It was found that these mice were protected from inflammatory arthritis by IVIG (FIG. 7c). Further, selective blockade of hDC-SIGN in transgenic hDC-SIGN⁺/SIGN-R1^{-/-} mice expressing both hDC-SIGN and hDC-SIGN-R resulted in a loss of IVIG protection *in vivo* (FIG. 7).

These results support a requirement for hDC-SIGN but not hDC-SIGN-R in this anti-inflammatory response triggered by sFc.

### Example 4: hDC-SIGN⁺ cells Transferred Anti-inflammatory Activity

In this example, assays were carried out to determine if stimulation of hDC-SIGN⁺ cells matured from bone marrow with sFc was sufficient to induce an anti-inflammatory response.

Bone marrow-derived macrophages (BMMΦ) and dendritic cells (BMDCs) cultured from hDC-SIGN⁺ transgenic 15 animals expressed hDC-SIGN, but not hDC-SIGN-R or SIGN-R1 (FIGs. 8a-c). Bone marrow-derived cells cultured from hDC-SIGN⁺ transgenic or WT mice were pulsed for 30 minutes with sFc or asialylated Fcs (asialoFc) at a concentration representative of *in vivo* treatments. The treated cells were collected, washed, and administered to WT mice, which were then challenged with K/BxN serum (FIG. 8d).

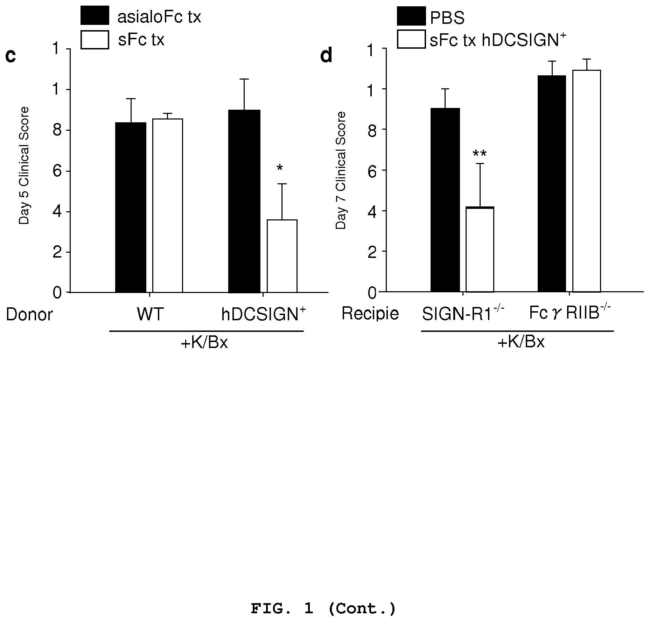

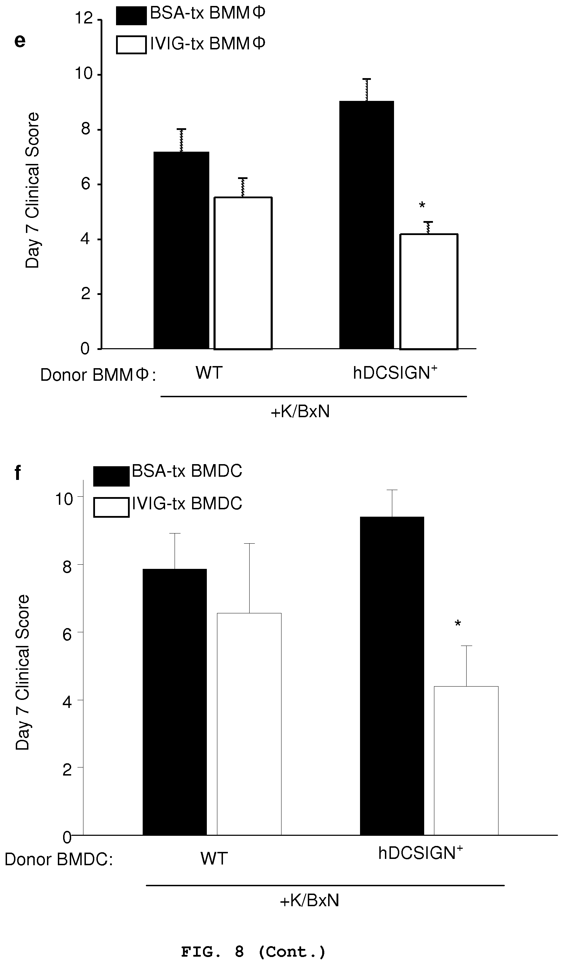

It was found that mice receiving hDC-SIGN⁺ BMMΦ or BMDCs pulsed with sFc or IVIG displayed reduced joint inflammation as compared to recipient mice that received WT cells, or hDC-SIGN⁺ cells pulsed with asialylated Fcs (asialoFc, FIG 1c, FIGs. 8e and f). It was also found that the anti-inflammatory response triggered by transferred sFc-stimulated hDC-SIGN⁺ BMMΦ required the expression of the inhibitory FcR, FcyRIIB, as FcγRIIB^{-/-} mice were not protected from inflammation induced by K/BxN serum (FIG. 1d). Collectively, these results were consistent with the *in vivo* requirements for IVIG protection previously defined (Nimmerjahn et al. Annu Rev Immunol 26, 513-533 (2008); Kaneko et al. Science 313, 670-673 (2006); Anthony et al. Science 320, 373-376 (2008); and Anthony et al. Proc Natl Acad Sci U S A13 105, 19571-19578 (2008)), and demonstrated that ligation of hDC-SIGN by sFc on bone marrow-derived myeloid cells is sufficient to induce an anti-inflammatory cellular response.

### Example 5: IL-4, but Not IL-10, Was Required for sFc's Anti-Inflammatory Activity

DC-SIGN engagement has been reported to result in dendritic cell production of IL-10 (Geijtenbeek et al. Nat Rev Immunol 9, 465-479 (2009); Gringhuis et al. Immunity 26, 605-24 616 (2007); and Hodges et al. Nat Immunol 27 8, 569-577 (2007)), making this anti-inflammatory cytokine an appealing candidate responsible for mediating IVIG anti-inflammatory activity. However, it was found that IL-10^{-/-} mice were protected from induced arthritis by IVIG similarly to wild type controls (FIG. 9). In this example, assays were carried out to identify other cytokines that could be responsible for this response.

The Th2 cytokine IL-4 has been shown to upregulate FcyRIIB surface expression on peripheral monocytes (Pricop et al. J Immunol 166, 531-537 (2001)), and increase the threshold for activation by pathogenic immune complexes, consistent with the FcyRIIB requirement of IVIG (Nimmerjahn et al. Annu Rev Immunol 26, 513-533 (2008); Bruhns et al. Immunity 18, 573-581 (2003); and Samuelsson et al. Science 291, 484-486 (2001)). Therefore, sFc-treated DC-SIGN⁺ BMMΦ were administered to naive WT mice or IL-4^{-/-} mice, and the recipient mice challenged with K/BxN serum. It was found that WT recipients were protected from induced arthritis, while IL-4^{-/-}recipients were not (FIG. 2a).

These results demonstrated that IVIG anti-inflammatory activity would require IL-4 signaling. Indeed, mice deficient in IL-4 (IL-4^{-/-}, FIG. 2b), the IL-4 receptor (IL-4Rα^{-/-}, FIG. 2c), and the IL-4R signaling adaptor (Stat6^{-/-}, FIG. 2c), were not protected from K/BxN-induced inflammation by IVIG or sFc. Further, it was found that monocytes in the peripheral blood and bone marrow of WT mice, but not IL-4Rα^{-/-} mice, upregulated FcyRIIB after sFc administration (FIG. 10).

### Example 6: IL-33 Triggered IL-4-Mediated Anti-Inflammatory Activity

In this example, assays were performed to examine whether exogenous Th2 cytokines could also suppress autoantibody-induced inflammation. Briefly, mice were treated with cytokine immune complexes (ic) (Finkelman et al. J Immunol 151, 1235-1244 (1993)) of Th2 cytokines IL-4 (IL-4ic), and IL-13 (IL-13ic), or a non-Th2 cytokine complex of IL-3 (IL-3ic), and challenged with K/BxN serum. It was found that inflammation was significantly attenuated following single administration of IL-4ic or IL-13ic, but not following IL-3ic treatment (FIG. 2d). However, IL-4ic treatment did not attenuate inflammation in FcγRIIB^{-/-} mice, consistent with IL-4ic also requiring the FcyRIIB to suppress inflammation (FIG. 2d).

It was examined whether Th2 cytokines were induced following IVIG or sFc administration. As shown in FIG. 3a and FIGs. 11a-c, no changes in IL-4 mRNA levels were observed. Assays then were performed to survey cytokines known to induce IL-4 expression, including IL-33 (Schmitz, J. et al. Immunity 23, 479-3 490 (2005); Neill et al. Nature 464, 1367-1370 (2010); and Paul et al. Nat Rev Immunol 10, 225-235 (2010)), IL-25 (Paul et al. Nat Rev Immunol 10, 225-235 (2010); Saenz et al. Nature 464, 1362-1366 (2010); and Fort et al. Immunity 15, 985-995 (2001)), and thymic stromal lymphopoietin (TSLP) (Paul et al. Nat Rev Immunol 10, 225-235 (2010); Soumelis et al. Nat Immunol 3, 673-680 (2002); and Wang et al. J Exp Med 204, 1837-1847 (2007)).

Interestingly, it was found that IL-33 mRNA was upregulated in WT mice following IVIG and sFc administration, but remained unchanged SIGNR1^{-/-} mice.

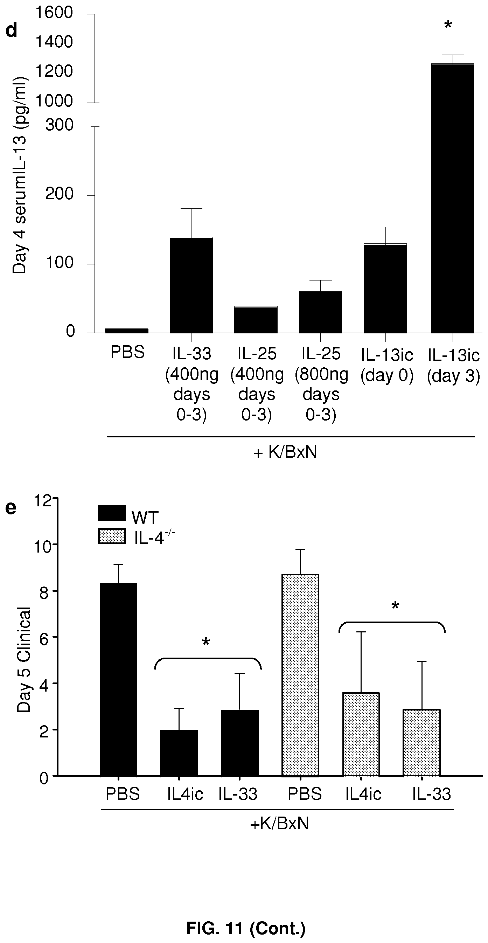

Then, IL-33, IL-25, or TSLP was administered to mice challenged with K/BxN serum. It was found that exogenous IL-33 fully suppressed K/BxN arthritogenic activity and induced IL-4 production *in vivo,* while IL-25 promoted only modest protection, and TSLP provided no protection (FIG. 3b), all of which correlated with systemic IL-4 and IL-13 levels (FIG. 3b and FIGs. 11d and e). IL-25 was reported to induce expansion of IL-13 expressing populations (Neill et al. immunity. Nature 464, 1367-1370 (2010); Moro et al. Nature 463, 540-544, (2010); and Price et al. Proc Natl Acad Sci USA 107, 11489-11494, (2010)). Low systemic levels of IL-13 were detected in IL-25 and K/BxN treated mice suggest that IL-13 is sequestered or not released during inflammation, and thus unable to increase FcyRIIB surface expression.

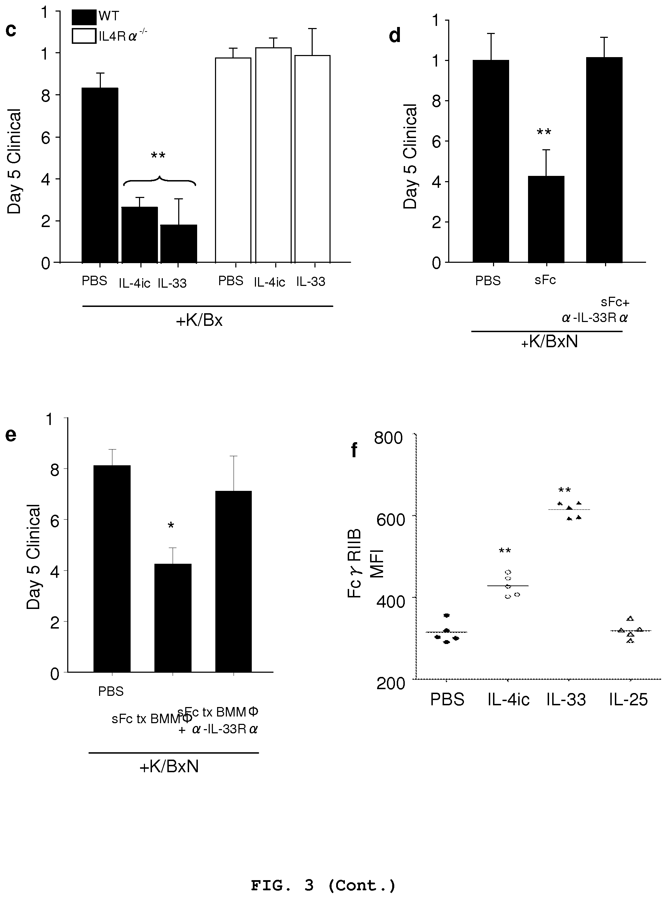

Further, it was found that exogenous IL-33 and IL-4ic were unable to ameliorate serum-induced arthritis in IL- 4Rα^{-/-} mice (FIG. 3c and FIG. 11e), suggesting IL-4Rα acts downstream of IL-33 in this pathway.

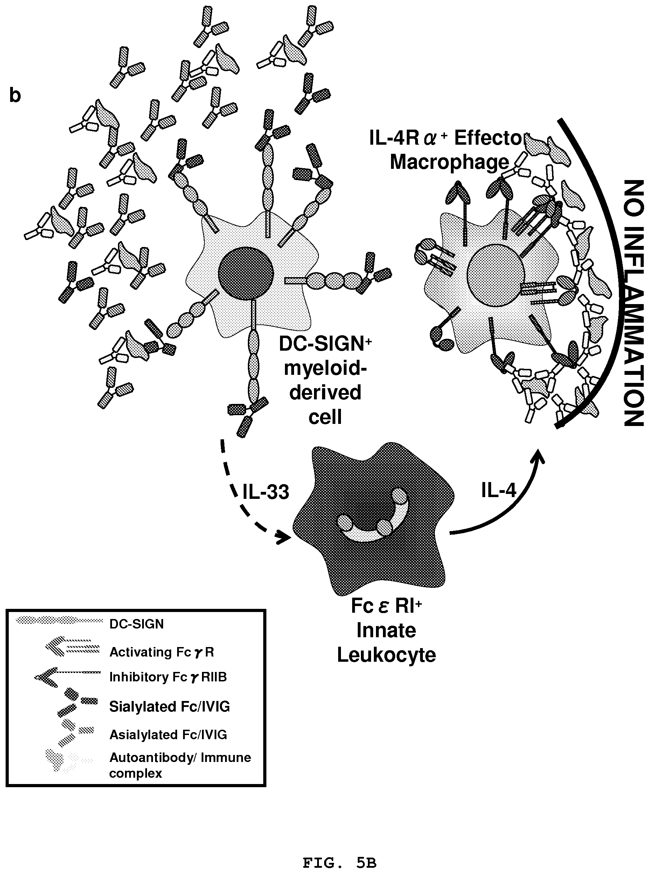

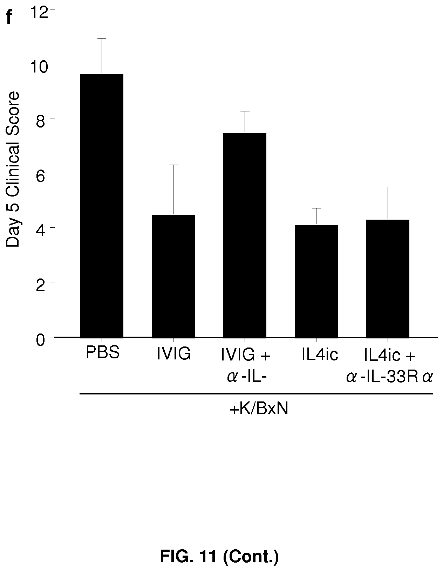

The above results support an anti-inflammatory cascade where DC-SIGN ligation by sFc promotes IL-33 production, IL-33 induces IL-4 expression, culminating in FcyRIIB upregulation on monocytes and macrophages (FIG. 5). To confirm this, hDC-SIGN⁺/SIGN-R1^{-/-} mice were treated with arthritogenic sera and sFc, in combination with a blocking antibody to the IL-33 receptor (α-IL-33Rα). It was found that this intervention ablated the ability of sFc to protect hDC21 SIGN⁺/SIGN-R1^{-/-} mice (FIG. 3d and FIG. 11f). Protection of transferred sFc treated hDC-SIGN⁺ BMMΦ was also diminished by α-IL-33Rα treatment (FIG. 3e). Further, it was found that administration of exogenous IL-4ic or IL-33 increased FcyRIIB surface expression on monocytes, while IL-25 had no effect (FIG. 3f and FIG. 12). IL-4 treatment downregulated FcyRIIB expression on B cells (FIG. 12), consistent with the diverse effects of this cytokine on different leukocyte types.

### Example 7: Basophils Mediated Anti-Inflammatory Activity

IL-4 can be produced by T cells and several innate immune cell populations (Paul et al. Nat Rev Immunol 10, 225-235 (2010)), including basophils (Min et al. J Exp Med 200, 507-517 (2004) and Seder et al. Proc Natl Acad Sci U S A 88, 2835-2839 (1991)), mast cells (Seder et al. Int Arch Allergy Appl Immunol 94, 24 137-140 (1991) and Wang et al. Clin Immunol 90, 27 47-54 (1999)), eosinophils (Shinkai et al. Nature 420, 825-829 (2002)), and progenitor cells (Saenz et al. Nature 464, 1362-1366 (2010)). IVIG activity is T cell independent (Anthony et al. Proc Natl Acad Sci U S A13 105, 19571-19578 (2008)), thus eliminating these cells as a source of sFc-induced IL-4. To determine if basophils were involved in this response, these cells were selectively depleted *in vivo* (Sokol et al. Nat Immunol 9, 310-318 (2008)) (FIG. 4a and FIGs. 13-15).

It was found that arthritis could be induced in basophil depleted hDC-SIGN⁺/SIGN R1^{-/-} mice (FIG. 13a) but the protective capacity of sFc and IVIG was lost (FIG. 4a and FIG. 14). These results suggested that basophils play a pivotal role.

Dendritic cells were reported to be affected by α-FcεRI treatment (Hammad et al. J Exp Med 207, 2097-2111, (2010). Therefore, dendritic cells (CD11^{c+} I-A^{b+}) were analyzed in α-FcεRI and isotype control treated mice. Histograms of gated dendritic cell populations expression of FcεRI are shown in FIG. 14, right column; α-FcεRI treated (gray histograms), isotype control treated (white histograms).

Next sFc and K/BxN sera were administered to IL-4-GFP reporter mice (4get; Mohrs et al. Immunity 15, 303-311 (2001)). As shown in FIG. 4b, a two-fold increase in GFP⁺ basophils (DX5⁺ FcεRI⁺ c-Kit₋) was found in the circulation of protected sFc-treated mice. This result indicated that basophils produced IL-4 in response to sFc.

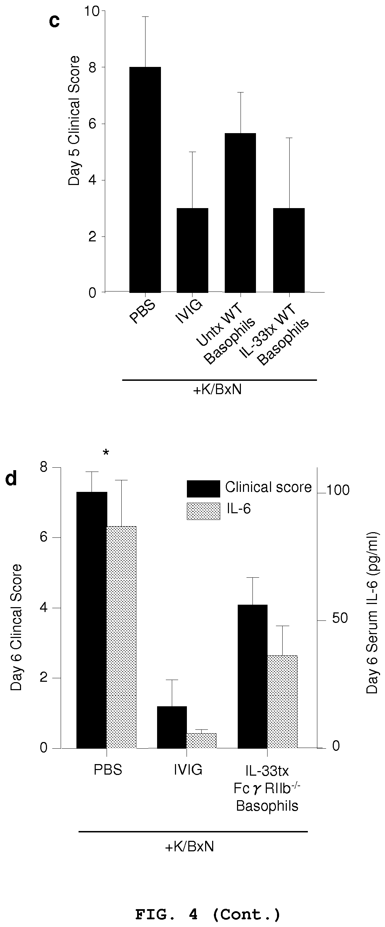

To determine whether basophils were ultimately responsible for the anti-inflammatory activity induced by sFc through IL-33, PBS or IL-33-treated basophils were transferred to K/BxN treated recipient mice (FIGs. 4c and d, and FIGs. 15a-c). More specifically, as shown in FIG. 16a, donor mice were treated with IL-3ic to expand basophils (Ohmori et al. J Immunol 182, 2835-2841, (2009). Five days later, the mice were administered PBS or IL-33. The next day (day 0), basophils (CD49b⁺ FcεRI⁺ c-Kit₋) were FACS sorted, and administered to naive recipient mice that then received K/BxN sera. As shown in the figures, IL-33-treated basophils derived from WT or FcγRIIB^{-/-} mice were equally effective at suppressing arthritic inflammation, reducing serum IL-6 levels, and curbing leukocyte infiltration to arthritic paws (FIG 4c, d; FIGs. 16d-e). These results confirm the anti-inflammatory potential of these cells, and support a model whereby IL-4 produced by basophils increases FcyRIIB expression on inflammatory macrophages (FIG. 5).

In sum, analyzing the anti-inflammatory activity of IVIG led to identification of an endogenous, innate pathway in which sialylated IgG, a minor component of serum IgG antibodies, bind DC-SIGN, promoting production of IL-33, which expands IL-4⁺ basophils. These cytokines are capable of suppressing autoantibody mediated inflammation by modulating FcyRIIB expression on effector cells (FIG. 5). IL-4 and IL-33 have pleiotropic activities, and mediate Th2 responses to helminth parasites and allergens (Samuelsson et al. Science 291, 484-486 (2001) and Paul et al. Nat Rev Immunol 10, 225-235 (2010)), as well as enhance inflammatory arthritis (Wang et al. Clin Immunol 90, 27 47-54 (1999) and Shinkai et al. Nature 420, 825-829 (2002)), in addition to their activities reported here. Cytokine concentration, cellular environment, and differential responses of individual cell types are likely to explain these distinct effector functions.

Various stimuli are reported to modulate the level of IgG Fc sialylation, and could regulate this intrinsic pathway. Antigenic stimulation results in production of pro-inflammatory, antigen-specific, asialylated IgG antibodies (Kaneko et al. Science 313, 670-673 (2006)). Pathogenic autoantibodies, such as those produced during rheumatoid arthritis recognizing citrullinated peptides, similarly show reduced sialic acid as compared to other serum antibodies (Scherer et al. Arthritis Rheum 62, 1620-1629 (2010)). Conversely, increases in sialylated IgG antibodies occur during pregnancy (van de Geijn et al. Arthritis Res Ther 11, R193 (2009)), which could contribute to the remission in arthritis seen in pregnant women. Therefore, affecting sialylation of IgG antibodies could provide an intrinsic mechanism for regulating Th2 cytokine production by innate myeloid cells in a DC-SIGN dependent manner, provide a means for maintaining homeostasis, and is an attractive therapeutic approach to suppressing inflammation in autoimmune diseases.

## Claims

1. A method for measuring the anti-inflammatory activity of a DC-SIGN-binding composition comprising a polypeptide containing a Fc region that has a N-linked biantennary oligosaccharide having a terminal sialic acid connected to galactose by an α 2,6 linkage comprising
(a) administering a DC-SIGN-binding composition comprising a polypeptide containing a Fc region that has a N-linked biantennary oligosaccharide having a terminal sialic acid connected to galactose by an α 2,6 linkage to a mouse; and
(b) measuring the expression level of IL-33 mRNA in splenocytes of the mouse, wherein an increase in the expression level of IL-33 mRNA in splenocytes of the mouse is indicative of the anti-inflammatory activity of the DC-SIGN-binding composition.

2. The method according to claim 1, wherein the DC-SIGN-binding composition is an IVIG preparation.

## Patentansprüche

1. Verfahren zur Messung der entzündungshemmenden Aktivität einer DC-SIGN-Bindungszusammensetzung, die ein Polypeptid umfasst, das eine Fc-Region enthält, die ein N-verknüpftes biantennäres Oligosaccharid aufweist, das eine durch eine α 2,6-Verknüpfung mit Galactose verbundene terminale Sialinsäure aufweist, umfassend
(a) Verabreichung einer DC-SIGN-Bindungszusammensetzung, die ein Polypeptid umfasst, das eine Fc-Region enthält, die ein N-verknüpftes biantennäres Oligosaccharid aufweist, das eine durch eine α 2,6-Verknüpfung mit Galactose verbundene terminale Sialinsäure aufweist, an eine Maus; und
(b) Messung des Expressionsniveaus von IL-33 mRNA in Splenozyten der Maus, wobei eine Erhöhung des Expressionsniveaus von IL-33 mRNA in Splenozyten der Maus die entzündungshemmende Aktivität der DC-SIGN-Bindungszusammensetzung anzeigt.

2. Verfahren nach Anspruch 1, wobei die DC-SIGN-Bindungszusammensetzung ein IVIG-Präparat ist.

## Revendications

1. Procédé de mesure de l'activité anti-inflammatoire d'une composition se liant à la DC-SIGN comprenant un polypeptide contenant une région Fc comportant un oligosaccharide biantennaire à liaison N comportant un acide sialique terminal relié au galactose par une liaison 2,6 α, comprenant
(a) l'administration d'une composition se liant à la DC-SIGN comprenant un polypeptide contenant une région Fc comportant un oligosaccharide biantennaire à liaison N comportant un acide sialique terminal relié au galactose par une liaison 2,6 α, à une souris ; et
(b) le mesure du niveau d'expression d'IL-33 mRNA en splénocytes du souris, une augmentation du niveau d'expression d'IL-33 mRNA en splénocytes de la souris indiquant l'activité anti-inflammatoire d'une composition se liant à la DC-SIGN.

2. Procédé selon la revendication 1, dans lequel la composition se liant à la DC-SIGN est une préparation d'IVIG.


### SEQUENCE LISTING

<110> THE ROCKEFELLER UNIVERSITY RAVETCH, Jeffrey V. ANTHONY, Robert M. KOBAYASHI, Toshihiko WERMELING, Fredrik
<120> ANTI-INFLAMMATORY AGENTS
<130> 070413.00133
<140> PCT/US12/33095
   <141> 2012-04-11
<150> 61/477,935
   <151> 2011-04-21
<160> 4
<170> PatentIn version 3.5
<210> 1
   <211> 270
   <212> PRT
   <213> Homo sapiens
<400> 1
   Met Lys Pro Lys Met Lys Tyr Ser Thr Asn Lys Ile Ser Thr Ala Lys
   1               5                   10                  15
   Trp Lys Asn Thr Ala Ser Lys Ala Leu Cys Phe Lys Leu Gly Lys Ser
               20                  25                  30
   Gln Gln Lys Ala Lys Glu Val Cys Pro Met Tyr Phe Met Lys Leu Arg
           35                  40                  45
   Ser Gly Leu Met Ile Lys Lys Glu Ala Cys Tyr Phe Arg Arg Glu Thr
       50                  55                  60
   Thr Lys Arg Pro Ser Leu Lys Thr Gly Arg Lys His Lys Arg His Leu
   65                  70                  75                  80
   Val Leu Ala Ala Cys Gln Gln Gln Ser Thr Val Glu Cys Phe Ala Phe
                   85                  90                  95
   Gly Ile Ser Gly Val Gln Lys Tyr Thr Arg Ala Leu His Asp Ser Ser
               100                 105                 110
   Ile Thr Gly Ile Ser Pro Ile Thr Glu Tyr Leu Ala Ser Leu Ser Thr
           115                 120                 125
   Tyr Asn Asp Gln Ser Ile Thr Phe Ala Leu Glu Asp Glu Ser Tyr Glu
       130                 135                 140
   Ile Tyr Val Glu Asp Leu Lys Lys Asp Glu Lys Lys Asp Lys Val Leu
   145                 150                 155                 160
   Leu Ser Tyr Tyr Glu Ser Gln His Pro Ser Asn Glu Ser Gly Asp Gly
                   165                 170                 175
   Val Asp Gly Lys Met Leu Met Val Thr Leu Ser Pro Thr Lys Asp Phe
               180                 185                 190
   Trp Leu His Ala Asn Asn Lys Glu His Ser Val Glu Leu His Lys Cys
           195                 200                 205
   Glu Lys Pro Leu Pro Asp Gln Ala Phe Phe Val Leu His Asn Met His
       210                 215                 220
   Ser Asn Cys Val Ser Phe Glu Cys Lys Thr Asp Pro Gly Val Phe Ile
   225                 230                 235                 240
   Gly Val Lys Asp Asn His Leu Ala Leu Ile Lys Val Asp Ser Ser Glu
                   245                 250                 255
   Asn Leu Cys Thr Glu Asn Ile Leu Phe Lys Leu Ser Glu Thr
               260                 265                 270
<210> 2
   <211> 266
   <212> PRT
   <213> Mus musculus
<400> 2
   Met Arg Pro Arg Met Lys Tyr Ser Asn Ser Lys Ile Ser Pro Ala Lys
   1               5                   10                  15
   Phe Ser Ser Thr Ala Gly Glu Ala Leu Val Pro Pro Cys Lys Ile Arg
               20                  25                  30
   Arg Ser Gln Gln Lys Thr Lys Glu Phe Cys His Val Tyr Cys Met Arg
           35                  40                  45
   Leu Arg Ser Gly Leu Thr Ile Arg Lys Glu Thr Ser Tyr Phe Arg Lys
       50                  55                  60
   Glu Pro Thr Lys Arg Tyr Ser Leu Lys Ser Gly Thr Lys His Glu Glu
   65                  70                  75                  80
   Asn Phe Ser Ala Tyr Pro Arg Asp Ser Arg Lys Arg Ser Leu Leu Gly
                   85                  90                  95
   Ser Ile Gln Ala Phe Ala Ala Ser Val Asp Thr Leu Ser Ile Gln Gly
   100 105 110
   Thr Ser Leu Leu Thr Gln Ser Pro Ala Ser Leu Ser Thr Tyr Asn Asp
   115 120 125
   Gln Ser Val Ser Phe Val Leu Glu Asn Gly Cys Tyr Val Ile Asn Val
   130 135 140
   Asp Asp Ser Gly Lys Asp Gln Glu Gln Asp Gln Val Leu Leu Arg Tyr
   145 150 155 160
   Tyr Glu Ser Pro Cys Pro Ala Ser Gln Ser Gly Asp Gly Val Asp Gly
   165 170 175
   Lys Lys Leu Met Val Asn Met Ser Pro Ile Lys Asp Thr Asp Ile Trp
   180 185 190
   Leu His Ala Asn Asp Lys Asp Tyr Ser Val Glu Leu Gln Arg Gly Asp
   195 200 205
   Val Ser Pro Pro Glu Gln Ala Phe Phe Val Leu His Lys Lys Ser Ser
   210 215 220
   Asp Phe Val Ser Phe Glu Cys Lys Asn Leu Pro Gly Thr Tyr Ile Gly
   225 230 235 240
   Val Lys Asp Asn Gln Leu Ala Leu Val Glu Glu Lys Asp Glu Ser Cys
   245 250 255
   Asn Asn Ile Met Phe Lys Leu Ser Lys Ile
   260 265
<210> 3
   <211> 153
   <212> PRT
   <213> Homo sapiens
<400> 3
<210> 4
   <211> 140
   <212> PRT
   <213> Mus musculus
<400> 4
   Met Gly Leu Asn Pro Gln Leu Val Val Ile Leu Leu Phe Phe Leu Glu
   1               5                   10                  15
   Cys Thr Arg Ser His Ile His Gly Cys Asp Lys Asn His Leu Arg Glu
               20                  25                  30
   Ile Ile Gly Ile Leu Asn Glu Val Thr Gly Glu Gly Thr Pro Cys Thr
           35                  40                  45
   Glu Met Asp Val Pro Asn Val Leu Thr Ala Thr Lys Asn Thr Thr Glu
       50                  55                  60
   Ser Glu Leu Val Cys Arg Ala Ser Lys Val Leu Arg Ile Phe Tyr Leu
   65                  70                  75                  80
   Lys His Gly Lys Thr Pro Cys Leu Lys Lys Asn Ser Ser Val Leu Met
                   85                  90                  95
   Glu Leu Gln Arg Leu Phe Arg Ala Phe Arg Cys Leu Asp Ser Ser Ile
               100                 105                 110
   Ser Cys Thr Met Asn Glu Ser Lys Ser Thr Ser Leu Lys Asp Phe Leu
           115                 120                 125
   Glu Ser Leu Lys Ser Ile Met Gln Met Asp Tyr Ser
       130                 135                 140